# EUROPÄISCHE PATENTSCHRIFT

(11) **EP 2 205 604 B1**
(45) Veröffentlichungstag und Bekanntmachung des Hinweises auf die Patenterteilung: **23.03.2011**
(21) Anmeldenummer: 08851418.7
(22) Anmeldetag: 16.10.2008
(51) Int. Cl.: C07D 487/04, A61K 31/5365, A61P 9/00, A61P 9/10, A61P 7/02

(54) **{ [5- ( PHENYL) - 6- PHENYLPYRROLO [2,1-F][2,1, 4]TRIAZIN-4-YL]AMINO} CARBONSÄUREDERIVATE UND VERWANDTE VERBINDUNGEN ALS PROSTAZYKLIN (PGI2) IP-REZEPTOR AKTIVATOREN ZUR BEHANDLUNG VON KARDIOVASKULÄREN ERKRANKUNGEN**
{[5-(PHENYL)-6-PHENYLPYRROLO[2,1-F][2,1,4]TRIAZIN-4-YL]AMINO}CARBOXYLIC ACID DERIVATIVES AND RELATED COMPOUNDS AS PROSTACYCLIN (PGI2) IP RECEPTOR ACTIVATORS FOR TREATING CARDIOVASCULAR DISORDERS
DÉRIVÉS DE L'ACIDE { [5- ( PHÉNYL) - 6- PHÉNYLPYRROLO [2,1-F][2,1, 4]TRIAZINE-4-YL]AMINO} CARBOXYLIQUE ET COMPOSÉS APPARENTÉS EN TANT QU'ACTIVATEURS DU RÉCEPTEUR DE PROSTAZYKLINE (PGI2) IP POUR LE TRAITEMENT DE MALADIES CARDIOVASCULAIRES

(30) Priorität: 30.10.2007 DE 102007051762
(43) Veröffentlichungstag der Anmeldung: 14.07.2010
(73) Patentinhaber: Bayer Schering Pharma Aktiengesellschaft, 13353 Berlin (DE)
(72) Erfinder: LAMPE, Thomas, 40545 Düsseldorf (DE); KAST, Raimund, 42349 Wuppertal (DE); STOLL, Friederike, 40215 Düsseldorf (DE); SCHUHMACHER, Joachim, 42113 Wuppertal (DE)
(86) Internationale Anmeldenummer: PCT/EP2008/008764
(87) Internationale Veröffentlichungsnummer: WO 2009/065472

(56) Entgegenhaltungen:
- WO-A-2006/047354
- WO-A-2007/079862

## Beschreibung

Die vorliegende Anmeldung betrifft neue, substituierte Pyrrolo[2,1-f][1,2,4]triazin-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Prostazyklin (PGI₂) gehört zur Familie der bioaktiven Prostaglandine, die Derivate der Arachidonsäure darstellen. PGI₂ ist das Hauptprodukt des Arachidonsäure-Stoffwechsels in Endothelzellen und hat potente gefäßerweiternde und anti-aggregatorische Eigenschaften. PGI₂ ist der physiologische Gegenspieler von Thromboxan A₂ (TxA₂), einem starken Vasokonstriktor und Stimulator der Thrombozytenaggregation, und trägt somit zur Aufrechterhaltung der vaskulären Homeostase bei. Eine Reduktion der PGI₂-Spiegel ist vermutlich mitverantwortlich für die Entstehung verschiedener kardiovaskulärer Erkrankungen [Dusting, G.J. et al., Pharmac. Ther. 1990, 48: 323-344; Vane, J. et al., Eur. J. Vasc. Endovasc. Surg. 2003, 26: 571-578].

Nach Freisetzung der Arachidonsäure aus Phospholipiden über Phospholipasen A₂ wird PGI₂ durch Cyclooxygenasen und anschließend durch die PGI₂-Synthase synthetisiert. PGI₂ wird nicht gespeichert, sondern nach Synthese sofort freigesetzt, wodurch es lokal seine Wirkungen entfaltet. PGI₂ ist ein instabiles Molekül, welches schnell (Halbwertszeit ca. 3 Minuten) nicht-enzymatisch zu einem inaktiven Metaboliten, 6-Keto-Prostaglandin-Flalpha, umgelagert wird [Dusting, G.J. et al., Pharmac. Ther. 1990, 48: 323-344].

Die biologischen Effekte von PGI₂ kommen durch die Bindung an einen membranständigen Rezeptor, den sogenannten Prostacyclin- oder IP-Rezeptor [Narumiya, S. et al., Physiol. Rev. 1999, 79: 1193-1226], zustande. Der IP-Rezeptor gehört zu den G-Protein-gekoppelten Rezeptoren, die durch sieben Transmembrandomänen charakterisiert sind. Neben dem humanen IP-Rezeptor sind auch noch die Prostacyclin-Rezeptoren aus Ratte und Maus kloniert worden [Vane, J. et al., Eur. J. Vasc. Endovasc. Surg. 2003, 26: 571-578]. In den Glattmuskelzellen führt die Aktivierung des IP-Rezeptors zur Stimulation der Adenylatzyklase, die die Bildung von cAMP aus ATP katalysiert. Die Erhöhung der intrazellulären cAMP-Konzentration ist für die Prostacyclin-induzierte Vasodilatation sowie die Hemmung der Thrombozytenaggregation verantwortlich. Neben den vasoaktiven Eigenschaften wurden für PGI₂ noch anti-proliferative [Schroer, K. et al., Agents Actions Suppl. 1997, 48: 63-91; Kothapalli, D. et al., Mol. Pharmacol. 2003, 64: 249-258; Planchon, P. et al., Life Sci. 1995, 57: 1233-1240] und anti-arteriosklerotische Wirkungen beschrieben [Rudic, R.D. et al., Circ. Res. 2005, 96: 1240-1247; Egan K.M. et al., Science 2004, 114: 784-794]. Darüber hinaus wird die Metastasenbildung durch PGI₂ gehemmt [Schneider, M.R. et al., Cancer Metastasis Rev. 1994, 13: 349-64). Ob diese Effekte durch Stimulation der cAMP-Bildung oder durch eine IP-Rezeptor-vermittelte Aktivierung anderer Signaltransduktionswege in der jeweiligen Zielzelle [Wise, H. et al. TIPS 1996, 17: 17-21], wie z.B. der Phosphoinositidkaskade sowie von Kaliumkanälen, zustande kommen, ist unklar.

Obwohl die Wirkungen von PGI₂ insgesamt therapeutisch von Nutzen sind, ist ein klinische Verwendung von PGI₂ durch seine chemische und metabolische Instabilität stark eingeschränkt. Stabilere PGI₂-Analoga wie z.B. Iloprost [Badesch, D.B. et al., J. Am. Coll. Cardiol. 2004, 43: 56S-61S] und Treprostinil [Chattaraj, S.C., Curr. Opion. Invest. Drugs 2002, 3: 582-586] konnten zwar zur Verfügung gestellt werden, allerdings ist die Wirkdauer dieser Verbindungen nach wie vor sehr kurz. Auch können die Substanzen nur über komplizierte Applikationswege dem Patienten verabreicht werden, wie z.B. durch Dauerinfusion, subkutan oder über mehrmalige Inhalationen. Diese Applikationswege können zudem zu zusätzlichen Nebenwirkungen, wie z.B. Infektionen oder Schmerzen an der Injektionsstelle, führen. Die Verwendung des bisher einzigen für den Patienten oral verfügbaren PGI₂-Derivates, Beraprost [Barst, R.J. et al., J. Am. Coll. Cardiol. 2003, 41: 2119-2125], ist wiederum durch seine kurze Wirkdauer limitiert.

Die Aufgabe der vorliegenden Erfindung liegt in der Bereitstellung neuer Substanzen, die als chemisch und metabolisch stabile, oral verfügbare Aktivatoren des IP-Rezeptors wirken und sich als solche zur Behandlung von Erkrankungen, insbesondere von kardiovaskulären Erkrankungen, eignen.

In WO 03/018589 werden 4-Aminofuro[2,3-d]pyrimidine als Adenosinkinase-Inhibitoren zur Behandlung kardiovaskulärer Erkrankungen beschrieben. Weitere 4-Amino-, 4-Oxy- oder 4-Thiosubstituierte Furo[2,3-d]pyrimidin-Derivate sowie ihre Verwendung zur Behandlung von kardiovaskulären Erkrankungen werden in WO 2007/079861 und WO 2007/079862 offenbart. Verbindungen mit Pyrrolo[2,1-f][1,2,4]triazin-Partialstruktur werden in WO 2006/004833 als Proteinkinase-Inhibitoren zur Behandlung von Krebserkrankungen beschrieben. In WO 2006/004884 werden Verfahren zur Herstellung bestimmter Pymolo[2,1-f][1,2,4]triazin-Derivate beansprucht.

Gegenstand der vorliegenden Erfindung sind Verbindungen der allgemeinen Formel (I) 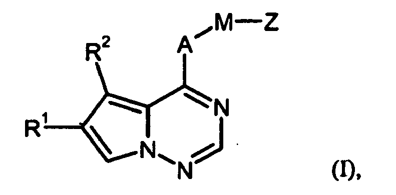 in welcher
- A: für O oder N-R³ steht, worin
R³ Wasserstoff, (C₁-C₆)-Alkyl, (C₃-C₇)-Cycloalkyl oder (C₄-C₇)-Cycloalkenyl bedeu- tet,
- M: für eine Gruppe der Formel 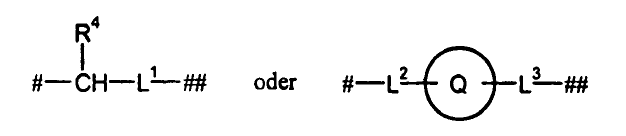 steht, worin
# die Verknüpfungstelle mit der Gruppe A und
## die Verknüpfungsstelle mit der Gruppe Z bedeuten,
R⁴ Wasserstoff oder (C₁-C₄)-Alkyl, welches mit Hydroxy oder Amino substituiert sein kann, bedeutet,
L¹ (C₁-C₇)-Alkandiyl oder (C₂-C₇)-Alkendiyl, welche ein- oder zweifach mit Fluor substituiert sein können, oder eine Gruppe der Formel *-L^{1A}-V-L^{1B}-** bedeu- tet, worin * die Verknüpfungsstelle mit der Gruppe -CHR⁴, ** die Verknüpfungsstelle mit der Gruppe Z, L^{1A} (C₁-C₅)-Alkandiyl, welches ein- oder zweifach, gleich oder verschieden, mit (C₁-C₄)-Alkyl und/oder (C₁-C₄)-Alkoxy substituiert sein kann, L^{1B} eine Bindung oder (C₁-C₃-Alkandiyl, welches ein- oder zweifach mit Fluor substituiert sein kann, und V O oder N-R⁵, worin R⁵ Wasserstoff, (C₁-C₆)-Alkyl oder (C₃-C₇)-Cycloalkyl bedeutet, darstellen,
L² eine Bindung oder (C₁-C₄)-Alkandiyl bedeutet,
L³ (C₁-C₄)-Alkandiyl bedeutet, welches ein- oder zweifach mit Fluor substituiert sein kann und in welchem eine Methylengruppe gegen O oder N-R⁶ ausgetauscht sein kann, worin R⁶ Wasserstoff, (C₁-C₆)-Alkyl oder (C₃-C₇)-Cycloalkyl darstellt, oder (C₂-C₄)-Alkendiyl bedeutet, und
Q (C₃-C₇)-Cycloalkyl, (C₄-C₇)-Cycloalkenyl, Phenyl, 5- bis 7-gliedriges Heterocyclyl oder 5- oder 6-gliedriges Heteroaryl bedeutet, welche jeweils bis zu zweifach, gleich oder verschieden, mit Resten ausgewählt aus der Reihe Fluor, Chlor, (C₁- C₄)-Alkyl, Trifluormethyl, Hydroxy, (C₁-C₄)-Alkoxy, Trifluormethoxy, Amino, Mono-(C₁-C₄)-alkylamino und Di-(C₁-C₄)-alkylamino substituiert sein können, wobei (C₁-C₄)-Alkyl seinerseits mit Hydroxy, (C₁-C₄)-Alkoxy, Amino, Mono- oder Di-(C₁-C₄)-alkylamino substituiert sein kann,
- Z: für eine Gruppe der Formel 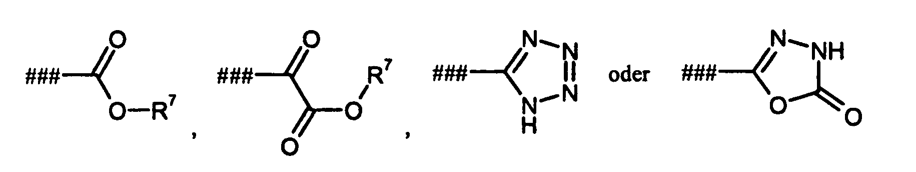 steht, worin
### die Verknüpfungsstelle mit der Gruppe L¹ beziehungsweise L³ und
R⁷ Wasserstoff oder (C₁-C₄)-Alkyl bedeutet, und
- R¹ und R²: gleich oder verschieden sind und unabhängig voneinander für (C₃-C₇)-Cycloalkyl, (C₄-C₇)-Cycloalkenyl, Phenyl, 5- bis 7-gliedriges Heterocyclyl oder 5- oder 6-gliedriges Heteroaryl stehen, welche jeweils ein- bis dreifach, gleich oder verschieden, mit Resten ausgewählt aus der Reihe Halogen, Cyano, Nitro, (C₁-C₆)-Alkyl, (C₁-C₆)-Alkenyl, (C₁-C₄)-Alkinyl, (C₃-C₇)-Cycloalkyl, (C₄-C₇)-Cycloalkenyl, (C₁-C₆)-Alkoxy, Trifluormethyl, Trifluormethoxy, (C₁-C₆)-Alkylthio, (C₁-C₆)-Acyl, Amino, Mono-(C₁-C₆)-alkylamino, Di-(C₁-C₆)-alkylamino und (C₁-C₆)-Acylamino substituiert sein können,
wobei (C₁-C₆)-Alkyl und (C₁-C₆)-Alkoxy ihrerseits jeweils mit Cyano, Hydroxy, (C₁-C₄)-Alkoxy, (C₁-C₄)-Alkylthio, Amino, Mono- oder Di-(C₁-C₄)-alkylamino substituiert sein können,
oder
- R¹ und/oder R²: für Phenyl stehen, worin zwei an benachbarte Ring-Kohlenstoffatome gebundene Reste zusammen eine Gruppe der Formel -O-CH₂-O-, -O-CHF-O-, -O-CF₂-O-, -O-CH₂-CH₂-O- oder -O-CF₂-CF₂-O- bilden,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Trisethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Insbesondere umfasst die vorliegende Erfindung bei den Verbindungen der Formel (I), in welcher
- Z: für eine Gruppe der Formel 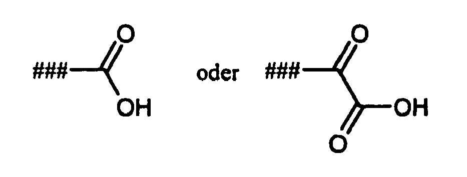 steht,
auch hydrolysierbare Ester-Derivate dieser Verbindungen. Hierunter werden Ester verstanden, die in physiologischen Medien, unter den Bedingungen der im weiteren beschriebenen biologischen Tests und insbesondere *in vivo* auf enzymatischem oder chemischem Wege zu den freien Carbonsäuren, als den biologisch hauptsächlich aktiven Verbindungen, hydrolysiert werden können. Als solche Ester werden (C₁-C₄)-Alkylester, in welchen die Alkylgruppe geradkettig oder verzweigt sein kann, bevorzugt. Besonders bevorzugt sind Methyl- oder Ethylester (siehe auch entsprechende Definitionen des Restes R⁷).

Im Rahmen der vorliegenden Erfindung haben die Substituenten, soweit nicht anders spezifiziert, die folgende Bedeutung:
(C₁-C₆)-Alkyl, (C₁-C₅)-Alkyl, (C₁-C₄)Alkyl und (C₁-C₃)-Alkyl stehen im Rahmen der Erfindung für einen geradkettigen oder verzweigten Alkylrest mit 1 bis 6, 1 bis 5, 1 bis 4 bzw. 1 bis 3 Kohlenstoffatomen. Bevorzugt ist ein geradkettiger oder verzweigter Alkylrest mit 1 bis 4, besonders bevorzugt mit 1 bis 3 Kohlenstoffatomen. Beispielhaft und vorzugsweise seien genannt: Methyl, Ethyl, n-Propyl, Isopropyl, n-Butyl, *iso*-Butyl, *sec*.-Butyl, *tert.*-Butyl, 1-Ethylpropyl, n-Pentyl und n-Hexyl.
(C₂-C₆)-Alkenyl, (C₂-C₅)-Alkenyl und (C₂-C₄)-Alkenyl stehen im Rahmen der Erfindung für einen geradkettigen oder verzweigten Alkenylrest mit 2 bis 6, 2 bis 5 bzw. 2 bis 4 Kohlenstoffatomen und einer oder zwei Doppelbindungen. Bevorzugt ist ein geradkettiger oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
(C₂-C₄)-Alkinyl steht im Rahmen der Erfindung für einen geradkettigen oder verzweigten Alkinylrest mit 2 bis 4 Kohlenstoffatomen und einer Dreifachbindung. Bevorzugt ist ein geradkettiger Alkinylrest mit 2 bis 4 Kohlenstoffatomen. Beispielhaft und vorzugsweise seien genannt: Ethinyl, n-Prop-1-in-1-yl, n-Prop-2-in-1-yl, n-But-2-in-1-yl und n-But-3-in-1-yl.
(C₁-C₄)-Alkandiyl und (C₁-C₃)-Alkandiyl stehen im Rahmen der Erfindung für einen geradkettigen oder verzweigten divalenten Alkylrest mit 1 bis 4 bzw. 1 bis 3 Kohlenstoffatomen. Bevorzugt ist jeweils ein geradkettiger Alkandiylrest mit 1 bis 4 bzw. 1 bis 3 Kohlenstoffatomen. Beispielhaft und vorzugsweise seien genannt: Methylen, Ethan-1,2-diyl (1,2-Ethylen), Ethan-1,1-diyl, Propan-1,3-diyl (1,3-Propylen), Propan-1,1-diyl, Propan-1,2-diyl, Propan-2,2-diyl, Butan-1,4-diyl (1,4-Butylen), Butan-1,2-diyl, Butan-1,3-diyl und Butan-2,3-diyl.
(C₁-C₇-Alkandiyl, (C₁-C₅)-Alkandiyl und (C₃-C₇-Alkandiyl stehen im Rahmen der Erfindung für einen geradkettigen oder verzweigten divalenten Alkylrest mit 1 bis 7, 1 bis 5 bzw. 3 bis 7 Kohlenstoffatomen. Bevorzugt ist jeweils ein geradkettiger Alkandiylrest mit 1 bis 7, 1 bis 5 bzw. 3 bis 7 Kohlenstoffatomen. Beispielhaft und vorzugsweise seien genannt: Methylen, Ethan-1,2-diyl (1,2-Ethylen), Ethan-1,1-diyl, Propan-1,3-diyl (1,3-Propylen), Propan-1,1-diyl, Propan-1,2-diyl, Propan-2,2-diyl, Butan-1,4-diyl (1,4-Butylen), Butan-1,2-diyl, Butan-1,3-diyl, Butan-2,3-diyl, Pentan-1,5-diyl (1,5-Pentylen), Pentan-2,4-diyl, 3-Methyl-pentan-2,4-diyl und Hexan-1,6-diyl (1,6-Hexylen).
(C₂-C₄)-Alkendiyl und (C₂-C₃)-Alkendiyl stehen im Rahmen der Erfindung für einen geradkettigen oder verzweigten divalenten Alkenylrest mit 2 bis 4 bzw. 2 bis 3 Kohlenstoffatomen und bis zu 2 Doppelbindungen. Bevorzugt ist jeweils ein geradkettiger Alkendiylrest mit 2 bis 4 bzw. 2 bis 3 Kohlenstoffatomen und einer Doppelbindung. Beispielhaft und vorzugsweise seien genannt: Ethen-1,1-diyl, Ethen-1,2-diyl, Propen-1,1-diyl, Propen-1,2-diyl, Propen-1,3-diyl, But-1-en-1,4-diyl, But-1-en-1,3-diyl, But-2-en-1,4-diyl und Buta-1,3-dien-1,4-diyl.
(C₂-C₇)-Alkendiyl und (C₃-C₇)-Alkendiyl stehen im Rahmen der Erfindung für einen geradkettigen oder verzweigten divalenten Alkenylrest mit 2 bis 7 bzw. 3 bis 7 Kohlenstoffatomen und bis zu 3 Doppelbindungen. Bevorzugt ist jeweils ein geradkettiger Alkendiylrest mit 2 bis 7 bzw. 3 bis 7 Kohlenstoffatomen und einer Doppelbindung. Beispielhaft und vorzugsweise seien genannt: Ethen-1,1-diyl, Ethen-1,2-diyl, Propen-1,1-diyl, Propen-1,2-diyl, Propen-1,3-diyl, But-1-en-1,4-diyl, But-1-en-1,3-diyl, But-2-en-1,4-diyl, Buta-1,3-dien-1,4-diyl, Pent-2-en-1,5-diyl, Hex-3-en-1,6-diyl und Hexa-2,4-dien-1,6-diyl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, *tert*.-Butoxy, n-Pentoxy und n-Hexoxy.
(C₁-C₆)-Alkylthio und (C₁-C₄)-Alkylthio stehen im Rahmen der Erfindung für einen geradkettigen oder verzweigten Alkylthiorest mit 1 bis 6 bzw. 1 bis 4 Kohlenstoffatomen. Bevorzugt ist ein geradkettiger oder verzweigter Alkylthiorest mit 1 bis 4 Kohlenstoffatomen. Beispielhaft und vorzugsweise seien genannt: Methylthio, Ethylthio, n-Propylthio, Isopropylthio, n-Butylthio, *tert*.-Butylthio, n-Pentylthio und n-Hexylthio.
(C₁-C₆)-Acyl [(C₁-C₆)-Alkanoyl], (C₁-C₅)-Acyl [(C₁-C₅)-Alkanoyl] und (C₁-C₄)-Acyl [(C₁-C₄)-Alkanoyl] stehen im Rahmen der Erfindung für einen geradkettigen oder verzweigten Alkylrest mit 1 bis 6, 1 bis 5 bzw. 1 bis 4 Kohlenstoffatomen, der in der 1-Position ein doppelt gebundenes Sauerstoffatom trägt und über die 1-Position verknüpft ist. Bevorzugt ist ein geradkettiger oder verzweigter Acylrest mit 1 bis 4 Kohlenstoffatomen. Beispielhaft und vorzugsweise seien genannt: Formyl, Acetyl, Propionyl, n-Butyryl, iso-Butyryl und Pivaloyl.
Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethyjamino, n-Propylamino, Isopropylamino und tert.-Butylamino.
Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N-*Dimethylamino, *N*,*N*-Diethylamino, *N*-Ethyl-*N*-methylamino, *N*-Methyl-*N*-n-propylamino, *N*-Isopropyl-*N*-n-propylamino, *N*-*tert*.-Butyl-*N*-methylamino, *N*-Ethyl-*N*-n-pentylamino und *N*-n-Hexyl-*N*-methylamino.
(C₁-C₂)-Acylamino und (C₁-C₄)-Acylamino stehen im Rahmen der Erfindung für eine Amino-Gruppe mit einem geradkettigen oder verzweigten Acyl-Substituenten, der 1 bis 6 bzw. 1 bis 4 Kohlenstoffatome aufweist und über die Carbonylgruppe verknüpft ist. Bevorzugt ist ein Acylamino-Rest mit 1 bis 4 Kohlenstoffatomen. Beispielhaft und vorzugsweise seien genannt: Formamido, Acetamido, Propionamido, n-Butyramido und Pivaloylamido.
(C₃-C₇)-Cycloalkyl, (C₃-C₆)-Cycloalkyl und (C₄-C₆)-Cycloalkyl stehen im Rahmen der Erfindung für eine monocyclische, gesättigte Cycloalkylgruppe mit 3 bis 7, 3 bis 6 bzw. 4 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.
(C₄-C₇)-Cycloalkenyl, (C₄-C₆)-Cycloalkenyl und (C₅-C₆)-Cycloalkenyl stehen im Rahmen der Erfindung für eine monocyclische Cycloalkylgruppe mit 4 bis 7, 4 bis 6 bzw. 5 oder 6 Kohlenstoffatomen und einer Doppelbindung. Bevorzugt ist ein Cycloalkenylrest mit 4 bis 6, besonders bevorzugt mit 5 oder 6 Kohlenstoffatomen. Beispielhaft und vorzugsweise seien genannt: Cyclobutenyl, Cyclopentenyl, Cyclohexenyl und Cycloheptenyl.
5- bis 7-gliedriges Heterocyclyl steht im Rahmen der Erfindung für einen gesättigten oder partiell ungesättigten Heterocyclus mit insgesamt 5 bis 7 Ringatomen, der ein oder zwei Ring-Heteroatome aus der Reihe N und/oder O enthält und über Ring-Kohlenstoffatome und/oder gegebenenfalls Ring-Stickstoffatome verknüpft ist. Bevorzugt ist ein 5- oder 6-gliedriger gesättigter Heterocyclus mit ein oder zwei Ring-Heteroatomen aus der Reihe N und/oder O. Beispielhaft seien genannt: Pyrrolidinyl, Pyrrolinyl, Pyrazolidinyl, Tetrahydrofuranyl, Piperidinyl, Piperazinyl, Dihydropyranyl, Tetrahydropyranyl, Morpholinyl, Hexahydroazepinyl und Hexahydro-1,4-diazepinyl. Bevorzugt sind Pyrrolidinyl, Tetrahydrofuranyl, Piperidinyl, Piperazinyl, Tetrahydropyranyl und Morpholinyl.
5- oder 6-gliedriges Heteroaryl steht im Rahmen der Erfindung für einen aromatischen Heterocyclus (Heteroaromaten) mit insgesamt 5 oder 6 Ringatomen, der ein oder zwei Ring-Heteroatome aus der Reihe N, O und/oder S enthält und über Ring-Kohlenstoffatome und/oder gegebenenfalls ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Pyridyl, Pyrimidinyl, Pyridazinyl und Pyrazinyl. Bevorzugt sind Thienyl, Pyridyl, Pyrimidinyl, Pyridazinyl und Pyrazinyl.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Besonders bevorzugt ist eine Substitution mit ein oder zwei gleichen oder verschiedenen Substituenten, ganz besonders bevorzugt die Substitution mit einem Substituenten.

Bevorzugt im Rahmen der vorliegenden Erfindung sind Verbindungen der Formel (I), in welcher
- A: für O oder NH steht,
- M: für eine Gruppe der Formel 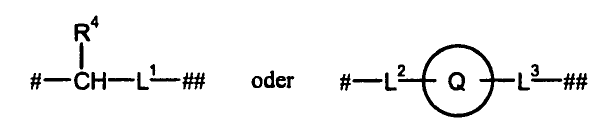 steht, worin
# die Verknüpfungstelle mit der Gruppe A und
## die Verknüpfungsstelle mit der Gruppe Z bedeuten,
R⁴ Wasserstoff, Methyl oder Ethyl bedeutet,
L¹ (C₃-C₇)-Alkandiyl, (C₃-C₇)-Alkendiyl oder eine Gruppe der Formel *-L^{1A}-V-L^{1B}-** bedeutet, worin * die Verknüpfungsstelle mit der Gruppe -CHR⁴,
** die Verknüpfungsstelle mit der Gruppe Z, L^{1A} (C₁-C₃)-Alkandiyl, welches ein- oder zweifach mit Methyl substituiert sein kann, L^{1B} (C₁-C₃)-Alkandiyl und V O oder N-CH₃ darstellen,
L² eine Bindung, Methylen, Ethan-1,1-diyl oder Ethan-1,2-diyl bedeutet,
L³ (C₁-C₃)-Alkandiyl oder eine Gruppe der Formel •-W-CH₂-•• oder •-W-CH₂-CH₂-•• bedeutet, worin • die Verknüpfungsstelle mit dem Ring Q, •• die Verknüpfungsstelle mit der Gruppe Z und W O oder N-R⁶, worin R⁶ Wasserstoff oder (C₁-C₃)-Alkyl bedeutet, darstellen,
und
Q Cyclobutyl, Cyclopentyl, Cyclopentenyl, Cyclohexyl, Pyrrolidinyl, Piperidinyl, Tetrahydrofuranyl, Tetrahydropyranyl, Morpholinyl oder Phenyl bedeutet, welche jeweils bis zu zweifach, gleich oder verschieden, mit Resten ausgewählt aus der Reihe Fluor, Methyl, Ethyl, Trifluormethyl, Hydroxy, Methoxy und Ethoxy substi- tuiert sein können,
- Z: für eine Gruppe der Formel 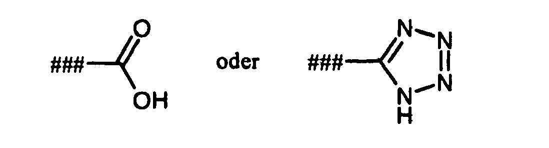 steht, worin
### die Verknüpfungsstelle mit der Gruppe L¹ beziehungsweise L³ bedeutet,
und
- R¹ und R²: gleich oder verschieden sind und unabhängig voneinander für Cyclopenten-1-yl, Cyclohexen-1-yl, Phenyl, Thienyl oder Pyridyl stehen, welche jeweils ein- oder zweifach, gleich oder verschieden, mit Resten ausgewählt aus der Reihe Fluor, Chlor, Cyano, (C₁-C₄)-Alkyl, (C₂-C₄)-Alkenyl, (C₁-C₄)-Alkoxy, Trifluormethyl und Trifluormethoxy substituiert sein können,
sowie ihre Salze, Solvate und Solvate der Salze.

Besonders bevorzugt im Rahmen der vorliegenden Erfindung sind Verbindungen der Formel (I), in welcher
- A: für O oder NH steht,
- M: für die Gruppe der Formel 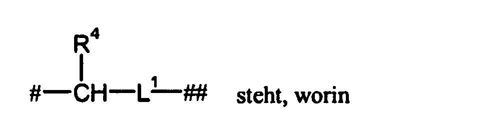
# die Verknüpfungstelle mit der Gruppe A
und
## die Verknüpfungsstelle mit der Gruppe Z bedeuten,
R⁴ Wasserstoff oder Methyl bedeutet,
und
L¹ Butan-1,4-diyl, Pentan-1,5-diyl oder eine Gruppe der Formel *-L^{1A}-O-L^{1B}-** bedeutet, worin * die Verknüpfungsstelle mit der Gruppe -CHR⁴, ** die Verknüpfungsstelle mit der Gruppe Z,
L^{1A} Methylen oder Ethan-1,2-diyl, welche ein- oder zweifach mit Methyl sub- stituiert sein können,
und
L^{1B} Methylen oder Ethan-1,2-diyl darstellen,
- Z: für die Gruppe der Formel 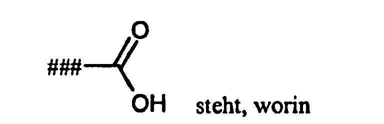
### die Verlrnüpfungsstelle mit der Gruppe L¹ bedeutet,
- R¹: für Phenyl steht, das mit Fluor oder Chlor substituiert sein kann,
und
- R²: für Phenyl steht, das mit Methyl, Ethyl, Methoxy oder Ethoxy substituiert sein kann,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Ganz besonders bevorzugt im Rahmen der vorliegenden Erfindung sind die im Folgenden genannten Verbindungen:
(6-{[5-(4-Methoxyphenyl)-6-phenylpyrrolo[2,1-f][1,2,4]triazin-4-yl]amino}hexansäure und
(6*R*)-6-{[5-(4-Methoxyphenyl)-6-phenylpyrrolo[2,1-f][1,2,4]triazin-4-yl]oxy} heptansäure sowie ihre Salze, Solvate und Solvate der Salze.

Weiterer Gegenstand der Erfindung ist ein Verfahren zur Herstellung der erfindungsgemäßen Verbindungen der Formel (I), in welcher Z für -COOH oder -C(=O)-COOH steht, dadurch gekennzeichnet, dass man entweder
[A] Verbindungen der Formel (II) 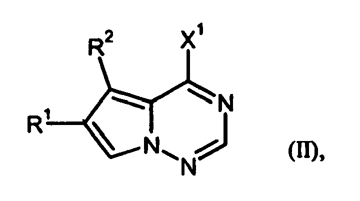 in welcher R¹ und R² die oben angegebenen Bedeutungen haben
   und
   X¹ für eine Abgangsgruppe wie beispielsweise Halogen, insbesondere für Chlor steht,
   in einem inerten Lösungsmittel in Gegenwart einer Base mit einer Verbindung der Formel (III) 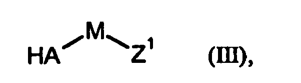 in welcher A und M die oben angegebenen Bedeutungen haben
   und
   Z¹ für Cyano oder eine Gruppe der Formel -[C(O)]_{y}-COOR^{7A} steht, worin
   - y: die Zahl 0 oder 1
   und
   R^{7A} (C₁-C₄)-Alkyl bedeutet,
   zu Verbindungen der Formel (IV) 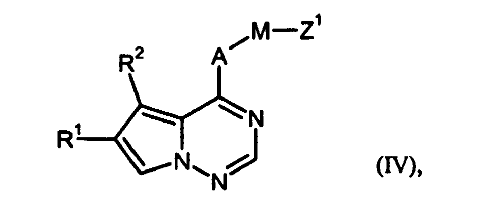 in welcher A, M, Z', R¹ und R² jeweils die oben angegebenen Bedeutungen haben, oder
[B] Verbindungen der Formel (V) 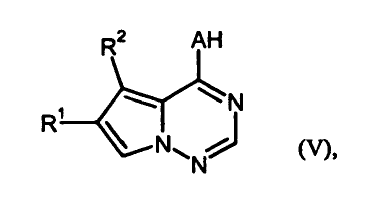 in welcher A, R¹ und R² jeweils die oben angegebenen Bedeutungen haben,
   in einem inerten Lösungsmittel in Gegenwart einer Base mit einer Verbindung der Formel (VI) 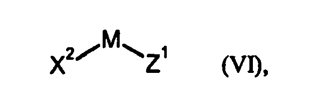 in welcher M und Z¹ die oben angegebenen Bedeutungen haben
   und
   - X²: für eine Abgangsgruppe wie beispielsweise Halogen, Mesylat, Tosylat oder Triflat steht,
   zu Verbindungen der Formel (IV) 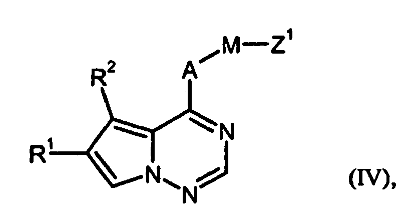 in welcher A, M, Z¹, R¹ und R² jeweils die oben angegebenen Bedeutungen haben, umsetzt
   und die Verbindungen der Formel (IV) dann durch Hydrolyse der Ester- bzw. Cyano-Gruppe Z¹ in die Carbonsäuren der Formel (I-A) 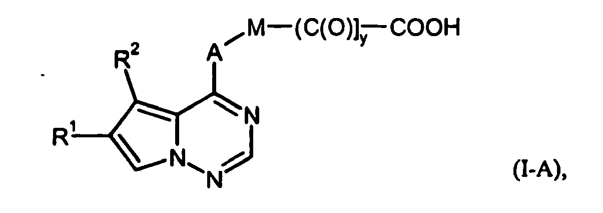 in welcher A, M, R¹, R² und y jeweils die oben angegebenen Bedeutungen haben,
   überführt und diese gegebenenfalls mit den entsprechenden (i) Lösungsmitteln und/oder (ii) Basen oder Säuren zu ihren Solvaten, Salzen und/oder Solvaten der Salze umsetzt.

Inerte Lösungsmittel für die Verfahrensschritte (II) + (III) → (IV) und (V) + (VI) → (IV) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethan, Tetrachlorethan, Trichlorethylen, Chlorbenzol oder Chlortoluol, oder andere Lösungsmittel wie Dimethylformamid (DMF), Dimethylsulfoxid (DMSO), *N*,*N'*-Dimethylpropylenharnstoff (DMPU), *N*-Methylpyrrolidon (NMP) oder Acetonitril. Ebenso ist es möglich, Gemische der genannten Lösungsmittel einzusetzen. Bevorzugt werden Tetrahydrofuran, Toluol, Dimethylformamid, Dimethylsulfoxid oder Gemische aus diesen verwendet.

Gegebenenfalls können die Verfahrensschritte (II) + (III) → (IV) und (V) + (VI) → (IV) jedoch auch ohne Lösungsmittel durchgeführt werden.

Als Basen für die Verfahrensschritte (II) + (III) → (IV) und (V) + (VI) → (IV) eignen sich übliche anorganische oder organische Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kalium-*tert*.-butylat, Alkalihydride wie Natrium- oder Kaliumhydrid, Amide wie Lithium- oder Kalium-bis(trimethylsilyl)amid oder Lithiumdiisopropylamid, metallorganische Verbindungen wie Butyllithium oder Phenyllithium, oder organische Amine wie Triethylamin, *N*-Methylmorpholin, *N-*Methylpiperidin, *N*,*N*-Diisopropylethylamin oder Pyridin.

Im Falle der Umsetzung mit Alkoholderivaten [A in (III) bzw. (V) = O] sind auch Phosphazen-Basen (so genannte "Schwesinger-Basen") wie beispielsweise P2-t-Bu oder P4-t-Bu zweckmäßig [vgl. z.B. R. Schwesinger, H. Schlemper, Angew. Chem. Int. Ed. Engl. 26, 1167 (1987); T. Pietzonka, D. Seebach, Chem. Ber. 124, 1837 (1991)].

Bei der Umsetzung mit Aminderivaten [A in (III) bzw. (V) = N] werden vorzugsweise tertiäre Amine, wie insbesondere *N*,*N*-Diisopropylethylamin, Natrium-tert.-butylat oder Natriumhydrid als Base verwendet. Gegebenenfalls können diese Umsetzungen aber auch - bei Verwendung eines Überschusses der Aminkomponente (III) - ohne Zusatz einer Hilfsbase erfolgen. Bei der Reaktion mit Alkoholderivaten [A in (III) bzw. (V) = O] sind Natriumhydrid, Kalium- oder Cäsiumcarbonat oder die Phosphazen-Basen P2-t-Bu und P4-t-Bu bevorzugt.

Die Verfahrensschritte (II) + (III) → (IV) und (V) + (VI) → (IV) können gegebenenfalls vorteilhaft unter Zusatz eines Kronenethers durchgerührt werden.

In einer Verfahrensvariante können die Reaktionen (II) + (III) → (IV) und (V) + (VI) → (IV) auch in einem Zwei-Phasen-Gemisch, bestehend aus einer wässrigen Alkalihydroxid-Lösung als Base und einem der oben genannten Kohlenwasserstoffe oder Halogenkohlenwasserstoffe als weiterem Lösungsmittel, unter Verwendung eines Phasentransfer-Katalysators wie Tetrabutylammoniumhydrogensulfat oder Tetrabutylammoniumbromid durchgeführt werden.

Die Verfahrensschritte (II) + (III) → (IV) und (V) + (VI) → (IV) erfolgen bei der Umsetzung mit Aminderivaten [A in (III) bzw. (V) = N] im Allgemeinen in einem Temperaturbereich von -20°C bis +150°C, bevorzugt bei 0°C bis +100°C. Bei der Umsetzung mit Alkoholderivaten [A in (III) bzw. (V) = O] werden die Reaktionen im Allgemeinen in einem Temperaturbereich von -20°C bis +120°C, bevorzugt bei -10°C bis +80°C durchgerührt.

Die Hydrolyse der Ester- bzw. Nitril-Gruppe Z¹ im Verfahrensschritt (IV) → (I-A) erfolgt nach üblichen Methoden, indem man die Ester bzw. Nitrile in inerten Lösungsmitteln mit Säuren oder Basen behandelt, wobei bei letzterem die zunächst entstehenden Salze durch Behandeln mit Säure in die freien Carbonsäuren überführt werden. Im Falle der *tert*.-Butylester erfolgt die Esterspaltung bevorzugt mit Säuren.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol, bei der Nitril-Hydrolyse bevorzugt Wasser und/oder n-Propanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von 0°C bis +100°C, bevorzugt bei +0°C bis +50°C. Die Nitril-Hydrolyse wird im Allgemeinen in einem Temperaturbereich von +50°C bis +150°C, bevorzugt bei +80°C bis +120°C durchgeführt.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Die erfindungsgemäßen Verbindungen der Formel (I), in welcher Z für eine Gruppe der Formel 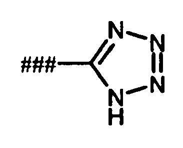 steht,
können hergestellt werden, indem man Verbindungen der Formel (IV), in welcher Z¹ für Cyano steht, in einem inerten Lösungsmittel mit einem Alkali-Azid in Gegenwart von Ammoniumchlorid oder mit Trimethylsilylazid gegebenenfalls in Gegenwart eines Katalysators umsetzt.

Inerte Lösungsmittel für diese Umsetzung sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Dimethylsulfoxid, Dimethylformamid, *N*,*N'*-Dimethylpropylenhamstoff (DMPU) oder *N-*Methylpyrrolidon (NMP). Ebenso ist es möglich, Gemische der genannten Lösungsmittel einzusetzen. Bevorzugt wird Toluol verwendet.

Als Azid-Reagenz ist insbesondere Natriumazid in Gegenwart von Ammoniumchlorid oder Trimethylsilylazid geeignet. Letztere Reaktion kann vorteilhafterweise in Gegenwart eines Katalysators durchgeführt werden. Hierfür eignen sich insbesondere Verbindungen wie Di-n-butylzinnoxid, Trimethylaluminium oder Zinkbromid. Bevorzugt wird Trimethylsilylazid in Kombination mit Din-butylzinnoxid verwendet.

Die Reaktion wird im Allgemeinen in einem Temperaturbereich von +50°C bis +150°C, bevorzugt bei +60°C bis +110°C durchgeführt. Die Umsetzung kann bei normalem, erhöhtem oder erniedrigtem Druck durchgeführt werden (z.B. von 0.5 bis 5 bar). Im Allgemeinen arbeitet man bei Normaldruck.

Die erfindungsgemäßen Verbindungen der Formel (I), in welcher Z für eine Gruppe der Formel 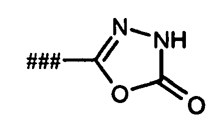 steht,
können hergestellt werden, indem man Verbindungen der Formel (IV), in welcher Z¹ für Methoxy- oder Ethoxycarbonyl [y = 0] steht, zunächst in einem inerten Lösungsmittel mit Hydrazin in Verbindungen der Formel (VII) 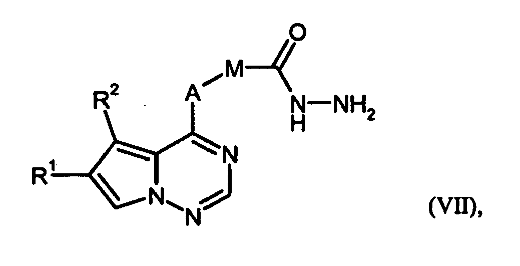 in welcher A, M, R¹ und R² jeweils die oben angegebenen Bedeutungen haben,
überführt und dann in einem inerten Lösungsmittel mit Phosgen oder einem Phosgen-Äquivalent, wie beispielsweise *N*,*N'*-Carbonyldiimidazol, umsetzt.

Als inerte Lösungsmittel sind für den ersten Schritt dieser Reaktionsfolge insbesondere Alkohole wie Methanol, Ethanol, n-Propanol, Isopropanol, n-Butanol oder *tert*.-Butanol, oder Ether wie Diethylether, Dioxan, Tetrahydrofuran, Glykoldimethylether oder Diethylenglykoldimethylether geeignet. Ebenso ist es möglich, Gemische dieser Lösungsmittel einzusetzen. Bevorzugt wird ein Gemisch aus Methanol und Tetrahydrofuran verwendet. Der zweite Reaktionsschritt wird vorzugsweise in einem Ether, insbesondere in Tetrahydrofuran durchgeführt. Die Umsetzungen erfolgen im Allgemeinen in einem Temperaturbereich von 0°C bis +70°C unter Normaldruck.

Die erfindungsgemäßen Verbindungen der Formel (I), in welcher L¹ für eine Gruppe der Formel *-L^{1A}-V-L^{1B}-** steht, worin L^{1A}, L^{1B} und V die oben angegebenen Bedeutungen haben, können alternativ auch dadurch hergestellt werden, dass man Verbindungen der Formel (VIII) 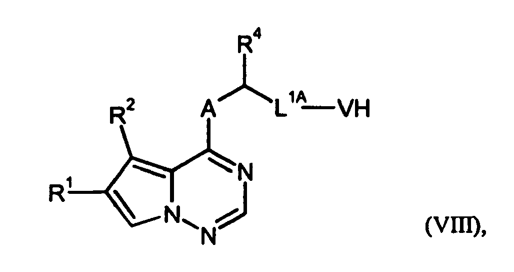 in welcher A, L^{1A}, V, R¹, R² und R⁴ jeweils die oben angegebenen Bedeutungen haben,
in Gegenwart einer Base gegebenenfalls in einem inerten Lösungsmittel mit einer Verbindung der Formel (IX) 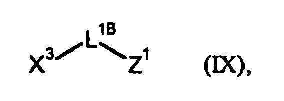 in welcher L^{1B} und Z¹ die oben angegebenen Bedeutungen haben
und
X³ für eine Abgangsgruppe wie beispielsweise Halogen, Mesylat, Tosylat oder Triflat steht, oder im Falle, dass L^{1B} für -CH₂CH₂- steht, mit einer Verbindung der Formel (X) 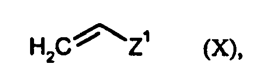 in welcher Z¹ die oben angegebene Bedeutung hat,
in Verbindungen der Formel (IV-A) 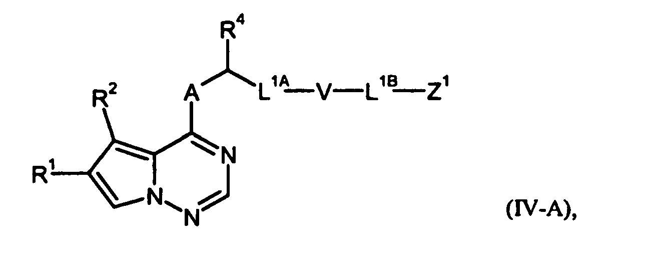 in welcher A, L^{1A}, L^{1B}, V, Z¹, R¹, R² und R⁴ jeweils die oben angegebenen Bedeutungen haben,
überführt und diese dann entsprechend dem zuvor beschriebenen Verfahren weiter umsetzt.

Die Verbindungen der Formel (VIII) können - analog zur Herstellung der Verbindungen (IV) - durch basenkatalysierte Reaktion einer Verbindung der Formel (II) oder (V) mit einer Verbindung der Formel (XI) bzw. (XII) 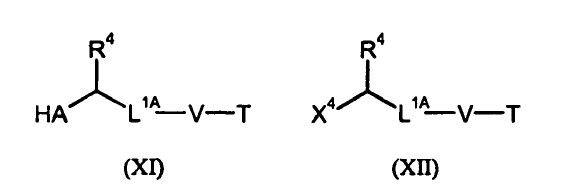 in welchen A, L^{1A}, V und R⁴ jeweils die oben angegebenen Bedeutungen haben,
T für Wasserstoff oder eine temporäre O- bzw. N-Schutzgruppe steht
und
X⁴ für eine Abgangsgruppe wie beispielsweise Halogen, Mesylat, Tosylat oder Triflat steht, erhalten werden (vgl. auch die nachfolgenden Reaktionsschemata 1 und 2).

In analoger Weise können die erfindungsgemäßen Verbindungen der Formel (I), in welcher L³ für eine Gruppe der Formel •-W-CH₂-•• oder •-W-CH₂-CH₂-•• steht, worin W die oben angegebene Bedeutung hat, auch dadurch hergestellt werden, dass man Verbindungen der Formel (XIII) 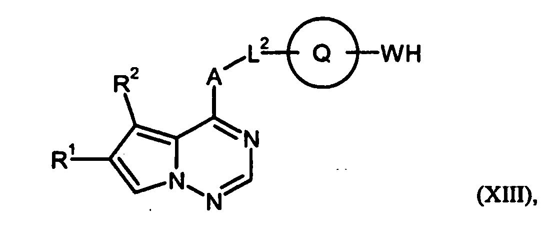 in welcher A, L², Q, W, R¹ und R² jeweils die oben angegebenen Bedeutungen haben,
in Gegenwart einer Base gegebenenfalls in einem inerten Lösungsmittel mit einer Verbindung der Formel (XIV) 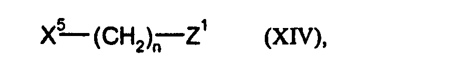 in welcher Z¹ die oben angegebene Bedeutung hat,
n für die Zahl 1 oder 2 steht
und
X⁵ für eine Abgangsgruppe wie beispielsweise Halogen, Mesylat, Tosylat oder Triflat steht, oder im Falle, dass L³ für •-W-CH₂CH₂-•• steht, mit einer Verbindung der Formel (X) 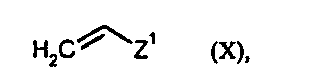 in welcher Z¹ die oben angegebene Bedeutung hat,
in Verbindungen der Formel (IV-B) 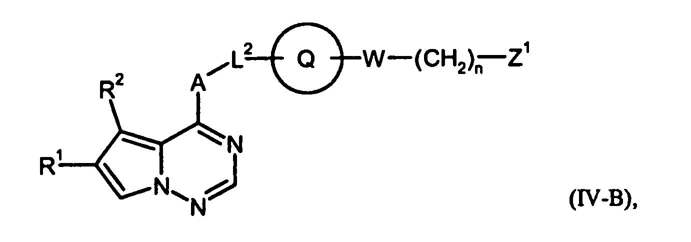 in welcher A, L², Q, W, Z¹, R¹, R² und n jeweils die oben angegebenen Bedeutungen haben,
überführt und diese dann entsprechend dem zuvor beschriebenen Verfahren weiter umsetzt.

Die Verbindungen der Formel (XIII) können - analog zur Herstellung der Verbindungen (IV) - durch basenkatalysierte Reaktion einer Verbindung der Formel (II) oder (V) mit einer Verbindung der Formel (XV) bzw. (XVI) 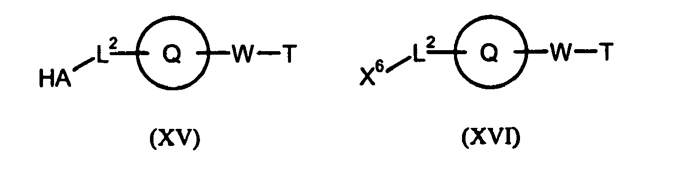 in welchen A, L², Q und W jeweils die oben angegebenen Bedeutungen haben,

T für Wasserstoff oder eine temporäre O- bzw. N-Schutzgruppe steht
und
X⁶ für eine Abgangsgruppe wie beispielsweise Halogen, Mesylat, Tosylat oder Triflat steht, erhalten werden (vgl. auch die nachfolgenden Reaktionsschemata 1 und 2).

Für die Verfahrensschritte (VIII) + (IX) bzw. (X) → (IV-A), (II) + (XI) → (VIII), (V) + (XII) → (VIII), (XIII) + (XIV) bzw. (X) → (IV-B), (II) + (XV) → (XIII) und (V) + (XVI) → (XIII) finden die zuvor für die Umsetzungen (II) + (III) → (IV) und (V) + (VI) → (IV) beschriebenen Reaktionsparameter wie Lösungsmittel, Basen und Reaktionstemperaturen in analoger Weise Anwendung.

Die Verbindungen der Formeln (II) und (V) können dadurch hergestellt werden, dass man 2-Cyanopyrrole der Formel (XVII) 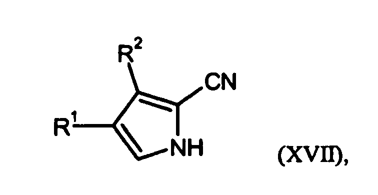 in welcher R¹ und R² die oben genannten Bedeutungen haben,
mit Hilfe eines Hydroxylamin-Derivats wie o-Mesitylensulfonylhydroxylamin oder (Aminooxy)-[bis(4-methoxyphenyl)]phosphinoxid [vgl. Smulik et al., Organic Letters 2003, 5 (22), 4187] in Gegenwart einer Base zu Verbindungen der Formel (XVIII) 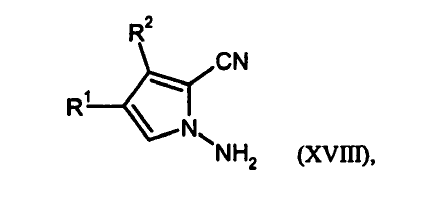 in welcher R¹ und R² die oben genannten Bedeutungen haben,
aminiert und diese dann entweder
[a] mit Formamid zu Verbindungen der Formel (V-A) 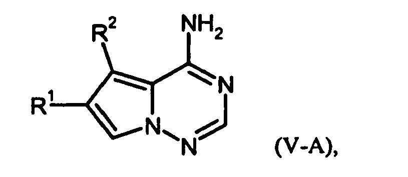 in welcher R¹ und R² die oben genannten Bedeutungen haben,
   kondensiert und gegebenenfalls nachfolgend mit Isoamylnitrit in Gegenwart einer Chlorid-Quelle wie Chlorwasserstoff oder Kupfer(II)chlorid zu Verbindungen der Formel (II-A) 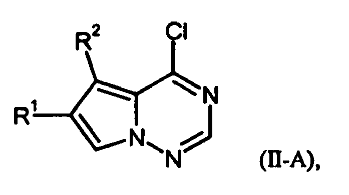 in welcher R¹ und R² die oben genannten Bedeutungen haben,
   umsetzt
   oder
[b] mit Ameisensäure in Gegenwart von Acetanhydrid zu Verbindungen der Formel (V-B) 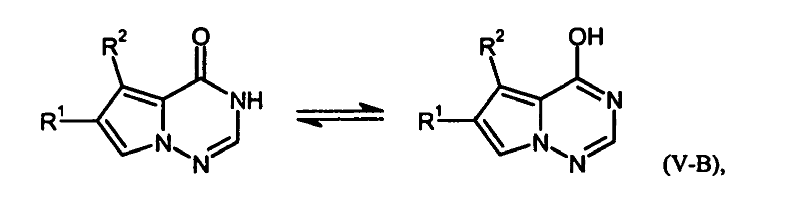 in welcher R¹ und R² die oben genannten Bedeutungen haben,
   kondensiert und gegebenenfalls nachfolgend mit Hilfe von Phosphomxychlorid in Verbindungen der Formel (II-A) überführt (siehe auch das nachfolgende Reaktionsschema 3).

Die Verbindungen der Formel (XVII) ihrerseits können beispielsweise dadurch hergestellt werden, dass man Verbindungen der Formel (XIX) 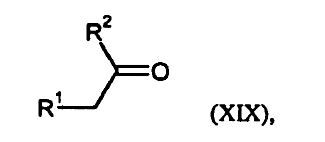 in welcher R¹ und R² die oben genannten Bedeutungen haben,
mit einem Dimethylformamid-Acetal zu Verbindungen der Formel (XX) 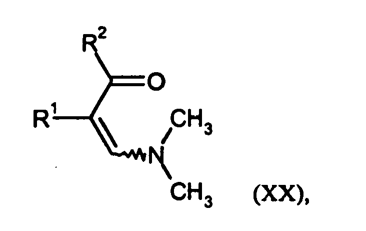 in welcher R¹ und R² die oben genannten Bedeutungen haben,
umsetzt und diese dann unter sauren Bedingungen mit 2-Amino-2-cyanoacetamid kondensiert (siehe auch das nachfolgende Reaktionsschema 3).

Die Verbindungen der Formeln (III), (VI), (IX), (X), (XI), (XII), (XIV), (XV), (XVI) und (XIX) sind kommerziell erhältlich, literaturbekannt oder können nach literaturbekannten Verfahren hergestellt werden.

Die Herstellung der erfindungsgemäßen Verbindungen kann durch die folgenden Syntheseschemata beispielhaft veranschaulicht werden:

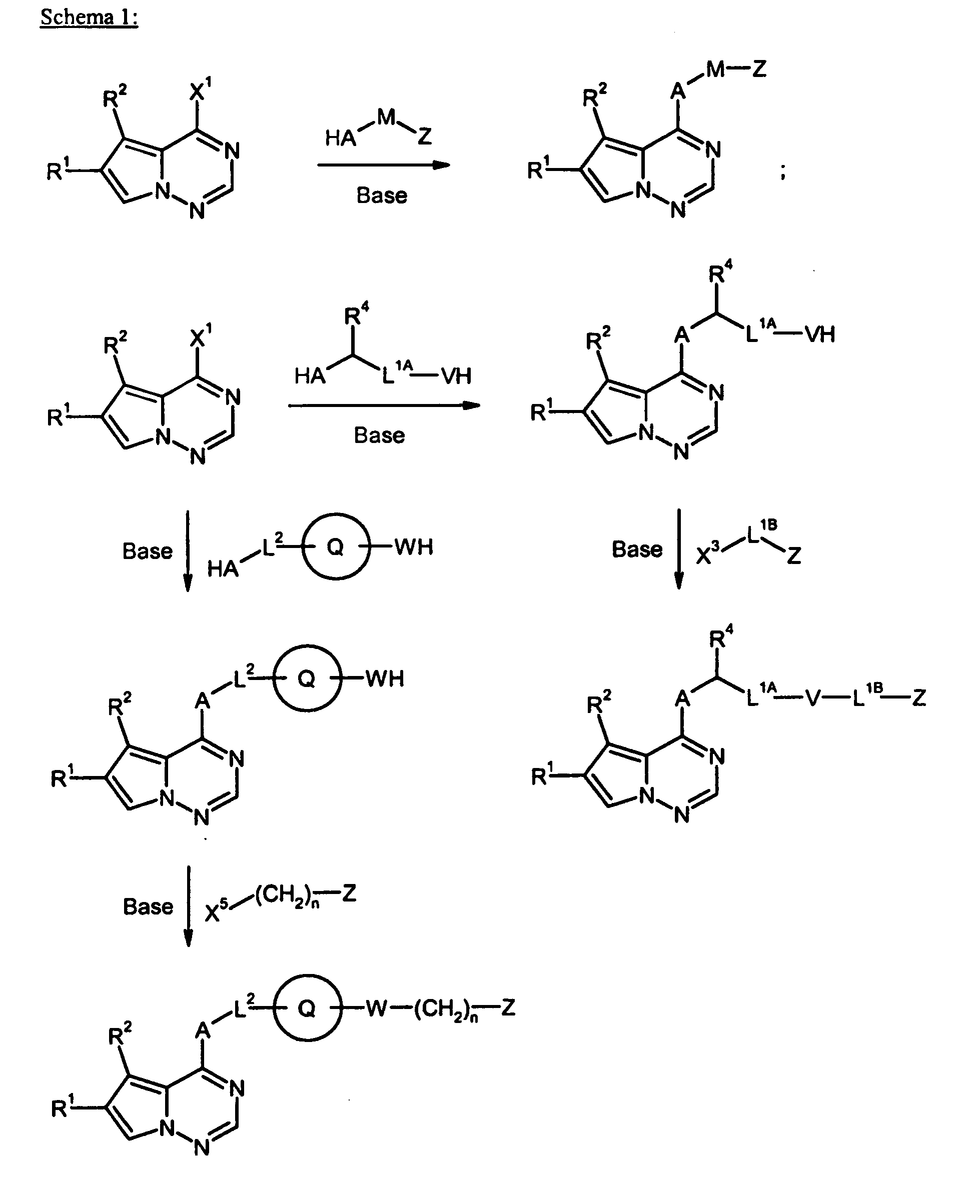

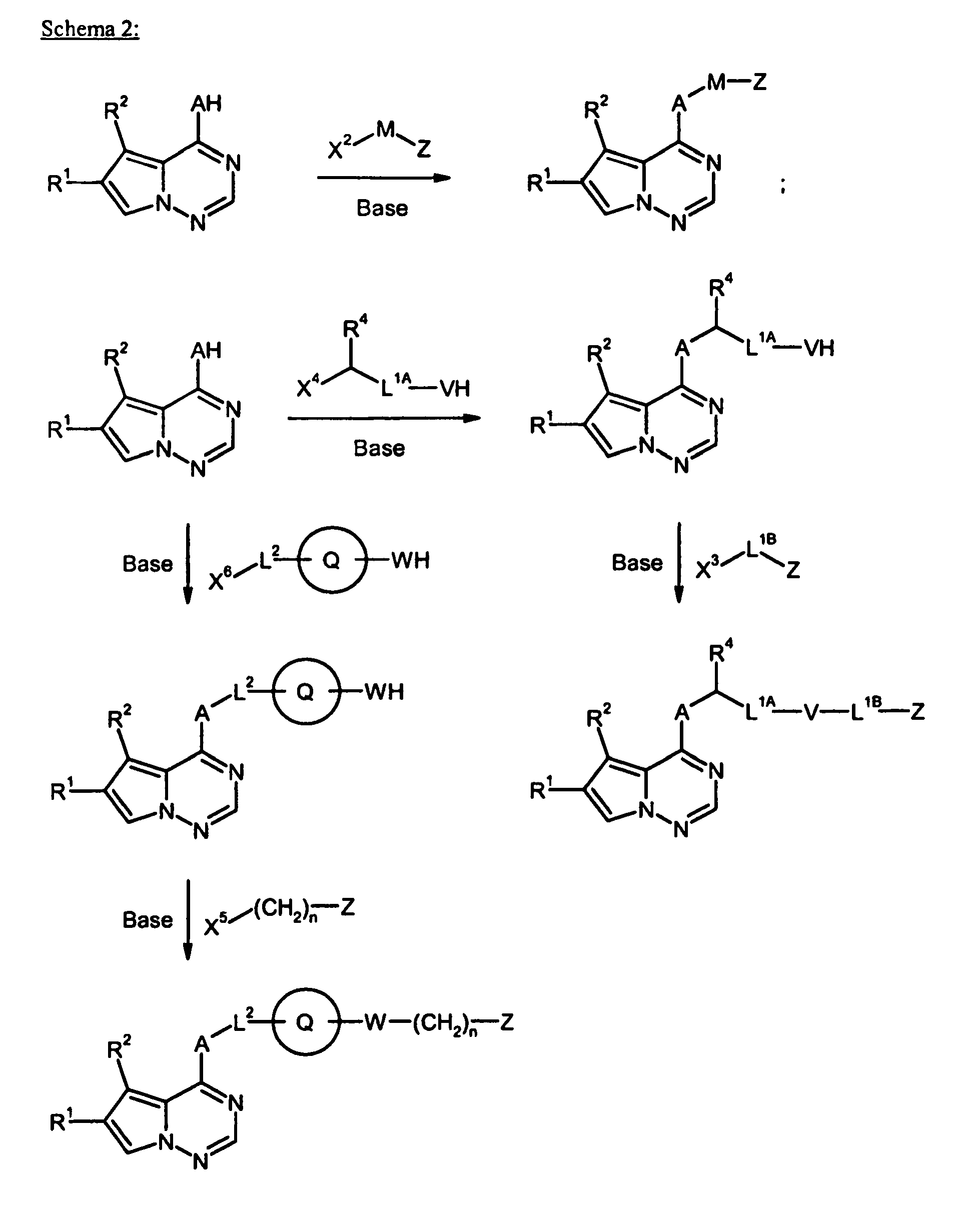

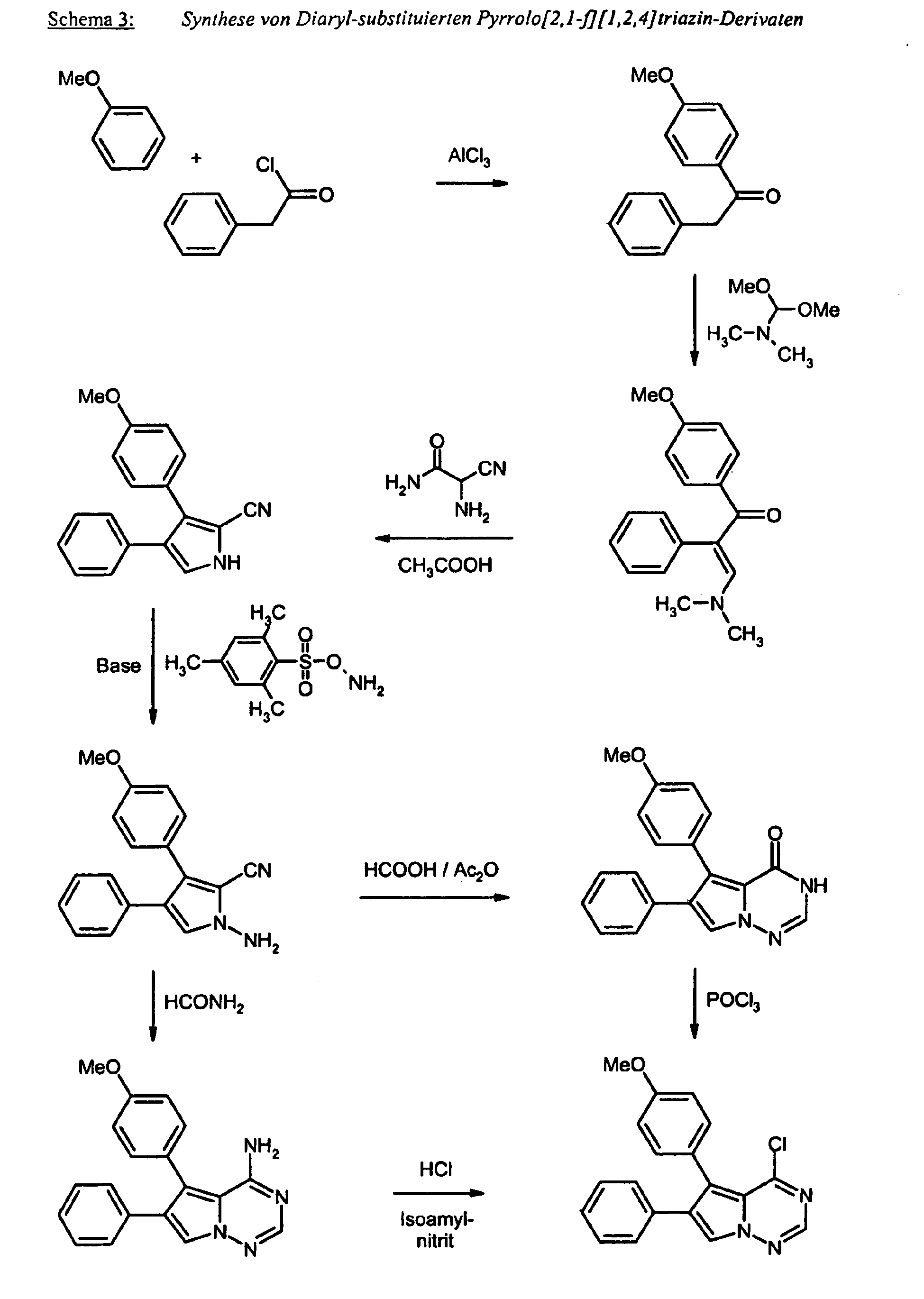

Die erfindungsgemäßen Verbindungen besitzen wertvolle pharmakologische Eigenschaften und können zur Vorbeugung und Behandlung von Erkrankungen bei Menschen und Tieren verwendet werden. Bei den erfindungsgemäßen Verbindungen handelt es sich um chemisch und metabolisch stabile, nicht-prostanoide Aktivatoren des IP-Rezeptors, die die biologische Wirkung von PGI₂ nachahmen.

Sie eignen sich damit insbesondere zur Prophylaxe und/oder Behandlung von kardiovaskulären Erkrankungen wie beispielsweise der stabilen und instabilen Angina pectoris, des Bluthochdrucks und der Herzinsuffizienz, der pulmonalen Hypertonie, zur Prophylaxe und/oder Behandlung von thromboembolischen Erkrankungen und Ischämien wie Myokardinfarkt, Hirnschlag, transitorischen und ischämischen Attacken sowie Subarachnoidalblutungen, und zur Verhinderung von Restenosen wie beispielsweise nach Thrombolysetherapien, percutan-transluminalen Angioplastien (PTA), Koronarangioplastien (PTCA) und Bypass.

Die erfindungsgemäßen Verbindungen sind insbesondere geeignet zur Behandlung und/oder Prophylaxe der pulmonalen Hypertonie (PH) einschließlich ihrer verschiedenen Ausprägungen. So eignen sich die erfindungsgemäßen Verbindungen in besonderem Maße zur Behandlung und/oder Prophylaxe der pulmonalen arteriellen Hypertonie (PAH) und deren Unterformen, wie der idiopathischen, der familiär bedingten und der beispielsweise mit portaler Hypertonie, fibrotischen Erkrankungen, HIV-Infektion oder unsachgemäßen Medikamentationen oder Toxinen assoziierten pulmonalen arteriellen Hypertonie.

Die erfindungsgemäßen Verbindungen können auch für die Behandlung und/oder Prophylaxe von anderen Formen der pulmonalen Hypertonie verwendet werden. So können sie beispielsweise für die Behandlung und/oder Prophylaxe der pulmonalen Hypertonie bei linksatrialen oder linksventrikulären Erkrankungen sowie bei linksseitigen Herzklappenerkrankungen eingesetzt werden. Darüber hinaus sind die erfindungsgemäßen Verbindungen zur Behandlung und/oder Prophylaxe der pulmonalen Hypertonie bei chronisch-obstruktiver Lungenkrankheit, interstitieller Lungenkrankheit, Lungenfibrose, Schlafapnoe-Syndrom, Erkrankungen mit alveolärer Hypoventilation, Höhenkrankheit und pulmonalen Entwicklungsstörungen geeignet.

Weiterhin eignen sich die erfindungsgemäßen Verbindungen zur Behandlung und/oder Prophylaxe der pulmonalen Hypertonie aufgrund chronischer thrombotischer und/oder embolischer Erkrankungen, wie beispielsweise Thromboembolie der proximalen Lungenarterien, Obstruktion der distalen Lungenarterien und Lungenembolie. Ferner können die erfindungsgemäßen Verbindungen zur Behandlung und/oder Prophylaxe der pulmonalen Hypertonie in Verbindung mit Sarkoidose, Histiozytosis X oder Lymphangioleiomyomatose sowie einer durch Gefäßkompression von außen (Lymphknoten, Tumor, fibrosierende Mediastinitis) bedingten pulmonalen Hypertonie verwendet werden.

Darüber hinaus können die erfindungsgemäßen Verbindungen auch zur Behandlung und/oder Prophylaxe von peripheren und kardialen Gefäßerkrankungen, von peripheren Verschlusskrankheiten (PAOD, PVD) sowie von peripheren Durchblutungsstörungen verwendet werden.

Ferner können die erfindungsgemäßen Verbindungen zur Behandlung von Arteriosklerose, Hepatitis, asthmatischen Erkrankungen, chronisch-obstruktiven Atemwegserkrankungen (COPD), Lungenödem, fibrosierenden Lungenerkrankungen wie idiopathische pulmonale Fibrose (IPF) und ARDS, entzündlichen vaskulären Erkrankungen wie Sklerodermie und Lupus erythematodes, von Nierenversagen, Arthritis und Osteoporose sowie zur Prophylaxe und/oder Behandlung von Krebserkrankungen, insbesondere von metastasierenden Tumoren, eingesetzt werden.

Außerdem können die erfindungsgemäßen Verbindungen auch als Zusatz zum Konservierungsmedium eines Organtransplantates, wie z.B. bei Nieren, Lungen, Herz oder Inselzellen, verwendet werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- NO- und Häm-unabhängige Aktivatoren der Guanylatcyclase, wie insbesondere die in WO 01/19355, WO 01/19776, WO 01/19778, WO 01/19780, WO 02/070462 und WO 02/070510 beschriebenen Verbindungen;
- Verbindungen, die die humane neutrophile Elastase (HNE) inhibieren, wie beispielsweise Sivelestat, DX-890 (Reltran), Elafin oder insbesondere die in WO 03/053930, WO 2004/020410, WO 2004/020412, WO 2004/024700, WO 2004/024701, WO 2005/080372, WO 2005/082863 und WO 2005/082864 beschriebenen Verbindungen;
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren;
- Verbindungen, die die lösliche Epoxidhydrolase (sEH) inhibieren, wie beispielsweise *N*,*N'-*Dicyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff;
- den Energiestoffwechsel des Herzens beeinflussende Verbindungen, wie beispielhaft und vorzugsweise Etomoxir, Dichloracetat, Ranolazine oder Trimetazidine;
- Agonisten von VPAC-Rezeptoren, wie beispielhaft und vorzugsweise das Vasoaktive Intestinale Polypeptid (VIP);
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Kinase-Inhibitor, wie beispielhaft und vorzugsweise Canertinib, Imatinib, Gefitinib, Erlotinib, Lapatinib, Lestaurtinib, Lonafamib, Pegaptinib, Pelitinib, Semaxanib, Tandutinib, Tipifarnib, Vatalanib, Sorafenib, Sunitinib, Bortezomib, Lonidamin, Leflunomid, Fasudil oder Y-27632, verabreicht.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095, SB-772077, GSK-269962A oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- abs.: absolut
- Ac: Acetyl
- Ac₂O: Acetanhydrid
- aq.: wässrig, wässrige Lösung
- c: Konzentration
- DC: Dünnschichtchromatographie
- DCI: direkte chemische Ionisation (bei MS)
- DIBAH: Düsobutylaluminiumhydrid
- DMF: *N,N*-Dimethylformamid
- DM SO: Dimethylsulfoxid
- d. Th.: der Theorie (bei Ausbeute)
- ee: Enantiomerenüberschuss
- EI: Elektronenstoß-Ionisation (bei MS)
- eq: Äquivalent(e)
- ESI: Elektrospray-Ionisation (bei MS)
- Fp.: Schmelzpunkt
- ges.: gesättigt
- h: Stunde(n)
- HPLC: Hochdruckflüssigchromatographie
- kat.: katalytisch
- konz.: konzentriert
- LC-MS: Flüssigchromatographie-gekoppelte Massenspektrometrie
- Me: Methyl
- min: Minute(n)
- MS: Massenspektrometrie
- NMR: Kernresonanzspektrometrie
- rac.: racemisch
- RP: reverse phase (Umkehrphase, bei HPLC)
- RT: Raumtemperatur
- R,: Retentionszeit (bei HPLC)
- TFA: Trifluoressigsäure
- THF: Tetrahydrofuran

### LC-MS-Methoden:

### Methode 1:

Gerätetyp MS: Micromass ZQ; Gerätetyp HPLC: HP 1100 Series; UV DAD; Säule: Phenomenex Gemini 3µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2:

Instrument: Micromass Quattro Premier mit Waters UPLC Acquity; Säule: Thermo Hypersil GOLD 1.9 µ, 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Ofen: 50°C; Fluss: 0.33 ml/min; UV-Detektion: 210 nm.

### Methode 3:

Gerätetyp MS: Micromass ZQ; Gerätetyp HPLC: Waters Alliance 2795; Säule: Phenomenex Synergi 2.5 µ MAX-RP 100A Mercury, 20 mm x 4 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 4:

Instrument: Micromass Quattro Micro MS mit HPLC Agilent Serie 1100; Säule: Thermo Hypersil GOLD 3µ, 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/rnin) → 5.00 min 100% A; Ofen: 50°C; Fluss: 2 ml/min; UV-Detektion: 210 nm.

### Ausgansverbindungen und Intermediate:

### Beispiel 1A

### (2E, 6R)-6-Hydroxyhept-2-ensäure-tert.-butylester

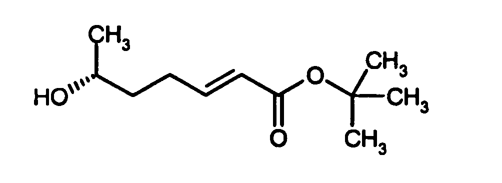

Lösung A: 10.71 g (267.7 mmol) 60%-iges Natriumhydrid wurden in 150 ml abs. THF suspendiert und tropfenweise unter Kühlung mit 43.3 ml (276.7 mmol) P,P-Dimethylphosphonoessigsäure-*tert*.-butylester versetzt. Die Mischung wurde bei RT gerührt, wobei nach ca. 30 min eine Lösung entstand.

Zu einer auf -78°C gekühlten Lösung von 17.87 g (178.5 mmol) (*R*)-γ-Valerolacton [(5*R*)-5-methyldihydrofuran-2(3*H*)-on] in 200 ml abs. THF wurden 187.4 ml (187.4 mmol) einer 1 M Lösung von DIBAH in THF getropft. Die Lösung wurde 1 h bei -78°C nachgerührt und dann die oben hergestellte Lösung A zugegeben. Nach Ende der Zugabe wurde die Mischung langsam auf RT erwärmt und über Nacht bei RT gerührt. Die Reaktionsmischung wurde zu 300 ml Ethylacetat gegeben und mit 50 ml konzentrierter Kalium-Natrium-Tartratlösung ausgerührt. Nach Phasentrennung wurde die wässrige Phase mit Ethylacetat re-extrahiert. Die organischen Phasen wurden vereinigt, mit ges. Natriumchlorid-Lösung gewaschen, über Magnesiumsulfat getrocknet und im Vakuum aufkonzentriert. Der Rückstand wurde mittels Chromatographie an Silicagel gereinigt (Laufmittel: Cyclohexan/Ethylacetat 5:1). Erhalten wurden 32.2 g (90.1 % d. Th.) des Zielprodukts, welches geringe Mengen des cis-Isomeren enthielt.
MS (DCI): m/z = 218 (M+NH₄)⁺
¹H-NMR (400 MHz, DMSO-d₆): δ = 6.70 (dt, 1H), 5.73 (d, 1H), 4.44 (d, 1H), 3.58 (m, 1H), 2.28-2.13 (m, 2H), 1.47-1.40 (m, 2H), 1.45 (s, 9H), 1.04 (d, 3H).

### Beispiel 2A

### (-)-6-Hydroxyheptansäure-tert.-butylester

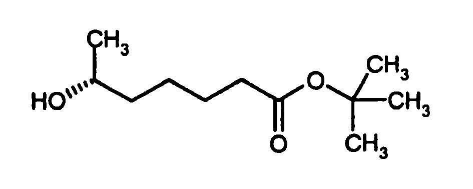

32.2 g (160.8 mmol) (2*E*,6*R*)-6-Hydroxyhept-2-ensäure-*tert*.-butylester wurden in 200 ml Ethanol gelöst und mit 1.7 g 10% Palladium auf Kohle versetzt. Die Mischung wurde bei RT unter einer Wasserstoffatmosphäre (Normaldruck) 2 h lang gerührt und dann über Celite abfiltriert. Das Filtrat wurde im Vakuum eingeengt. Aus dem Rückstand erhielt man nach Chromatographie an Silicagel (Laufmittel: Cyclohexan/Ethylacetat 10:1 → 6:1) 15.66 g des Zielprodukts (48.1 % d. Th.).
MS (DCI): m/z = 220 (M+NH₄)⁺
¹H-NMR (400 MHz, CDCl₃): δ = 3.85-3.75 (m, 1H), 2.22 (t, 2H), 1.68-1.54 (m, 2H), 1:53-1.30 (m, 4H), 1.45 (s, 9H), 1.18 (d, 3H).
[α]_{D}²⁰ = -21°, c = 0.118, Chloroform.

### Beispiel 3A

### 1-(4-Methoxyphenyl)-2-phenylethanon

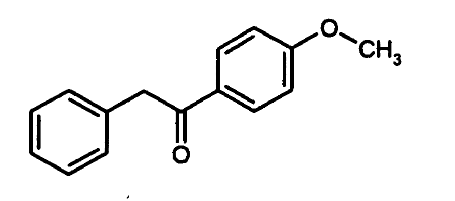

10.0 g (64.7 mmol) Phenylessigsäurechlorid wurden bei 0°C zu einer Suspension von 9.86 g (73.9 mmol) Aluminiumtrichlorid in 200 ml 1,2-Dichlorethan getropft. Die Mischung wurde 5 min bei 0°C gerührt, bevor tropfenweise 6.66 g (61.6 mmol) Anisol zugegeben wurden (Innentemperatur 5-8°C). Nach Ende der Zugabe wurde die Kühlung entfernt und die Mischung 2.5 h bei RT gerührt. Danach wurde der Ansatz unter kräftigem Rühren auf Eiswasser gegeben. Nach Zugabe von konz. Salzsäure wurde mit 1,2-Dichlorethan extrahiert. Die organische Phase wurde nacheinander mit Wasser, 1 N Natronlauge und ges. Natriumchlorid-Lösung gewaschen, über Natriumsulfat getrocknet und im Vakuum eingeengt. Der Rückstand wurde im Hochvakuum getrocknet. Erhalten wurden 16.24 g des Zielprodukts, welches ohne weitere Aufreinigung in der Folgereaktion eingesetzt wurde.
LC-MS (Methode 1): Rₜ = 2.50 min; m/z = 227 (M+H)⁺
¹H-NMR (400 MHz, DMSO-d₆): δ = 8.04 (d, 2H), 7.35-7.20 (m, 5H), 7.05 (d, 2H), 4.32 (s, 2H), 3.86 (s, 3H).

### Beispiel 4A

### 3-(Dimethylamino)-1-(4-methoxyphenyl)-2-phenylprop-2-en-1-on

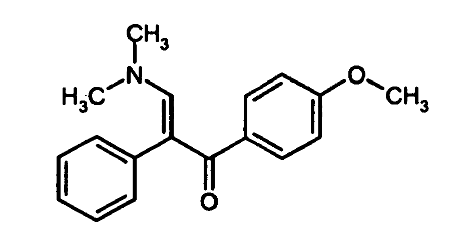

21.7 g (95.9 mmol) 1-(4-Methoxyphenyl)-2-phenylethanon wurden in 110 ml Toluol gelöst, auf 50°C erwärmt und mit 19.1 ml (143.9 mmol) *N,N*-Dimethylformamid-dimethylacetal versetzt. Die Reaktionsmischung wurde über Nacht bei 80°C gerührt und dann nach Abkühlen im Vakuum eingeengt. Der Rückstand wurde mehrmals nacheinander in Toluol aufgenommen und jeweils wieder im Vakuum zur Trockene eingeengt. Der anfallende Feststoff wurde in Petrolether verrührt, abfiltriert und im Hochvakuum getrocknet. Erhalten wurden 24.26 g des Zielprodukts (89.9% d. Th.).
¹H-NMR (400 MHz, DMSO-d₆): δ = 7.37 (d, 2H), 7.31-7.18 (m, 4H), 7.10 (d, 2H), 6.87 (d, 2H), 3.28 (s, 3H), 2.68 (s, 6H).

### Beispiel 5A

### 3-(4-Methoxyphenyl)-4-phenyl-1H-pyrrol-2-carbonitril

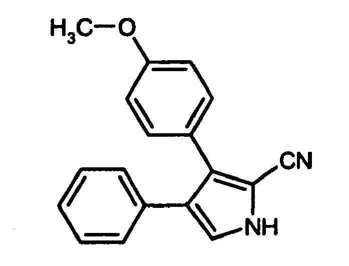

Zu einer Lösung von 24.0 g (85.3 mmol) 3-(Dimethylamino)-1-(4-methoxyphenyl)-2-phenylprop-2-en-1-on in 144 ml Eisessig wurden 8.45 g (85.3 mmol) 2-Amino-2-cyanoacetamid gegeben. Die Reaktionsmischung wurde 1 h auf 80°C erhitzt. Nach Abkühlen auf RT wurden 5 ml konz. Schwefelsäure zugesetzt und die Reaktionsmischung eine weitere Stunde bei RT gerührt. Man gab dann den Ansatz auf Wasser und extrahierte mit Dichlormethan. Die organische Phase wurde mit Wasser, ges. Natriumhydrogencarbonat-Lösung und ges. Natriumchlorid-Lösung gewaschen, über Natriumsulfat getrocknet und im Vakuum eingeengt. Das Rohprodukt wurde durch Chromatographie an Silicagel gereinigt (Laufmittel: Cyclohexan/Ethylacetat 6:1 → 4:1). Erhalten wurden 13.49 g des Zielprodukts (49.2% d. Th.).
LC-MS (Methode 1): Rₜ = 2.65 min; m/z = 275 (M+H)⁺
¹H-NMR (400 MHz, DMSO-d₆): δ = 12.53 (s, 1H), 7.41 (s, 1H), 7.29-7.15 (m, 7H), 6.94 (d, 2H), 3.80 (s, 3H).

### Beispiel 6A

### 1-Amino-3-(4-methoxyphenyl)-4-phenyl-1H-pyrrol-2-carbonitril

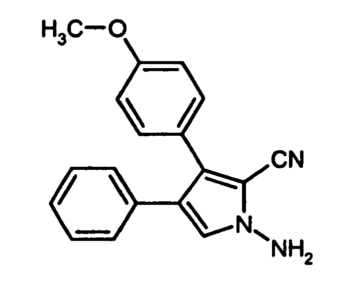

Unter einer Argonatmosphäre wurde zu einer eisgekühlten Suspension von 700 mg (17.5 mmol, 60%-ig) Natriumhydrid in 30 ml DMF eine Lösung von 960 mg (2.5 mmol) 3-(4-Methoxyphenyl)-4-phenyl-1*H-*pyrrol-2-carbonitril in 3.4 ml DMF getropft. Die Mischung wurde 10 min bei 0°C gerührt. Anschließend wurde eine Lösung von 1130 mg (ca. 5 mmol) o-Mesitylensulfonylhydroxylamin (noch leicht wasserfeucht, frisch hergestellt gemäß *Synthesis,* **1972, 140**; Vorsicht: kann im trockenen Zustand explosiv sein!) in ca. 5 ml DMF zugegeben. Die Reaktionsmischung wurde 3 h bei 0°C gerührt, dann mit Ethylacetat verdünnt und vorsichtig mit ges. Ammoniumchlorid-Lösung versetzt. Die organische Phase wurde mit ges. Natriumchlorid-Lösung gewaschen, über Natriumsulfat getrocknet und im Vakuum eingeengt. Der Rückstand wurde durch Chromatographie an Silicagel gereinigt (Laufmittel: Cyclohexan/Ethylacetat 7:1). Es wurden 594 mg des Zielprodukts erhalten (58.7% d. Th.).
LC-MS (Methode 2): Rₜ = 1.23 min; m/z = 290 (M+H)⁺
¹H-NMR (400 MHz, DMSO-d₆): δ = 7.31 (s, 1H), 7.29-7.11 (m, 7H), 6.95 (d, 2H), 6.49 (s, 2H), 3.79 (s, 3H).

### Beispiel 7A

### 4-Amino-5-(4-methoxyphenyl)-6-phenylpyrrolo[2,1-f][1,2,4]triazin

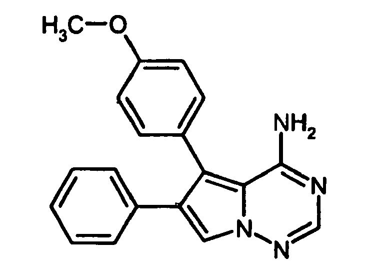

590 mg (2.04 mmol) 1-Amino-3-(4-methoxyphenyl)-4-phenyl-1*H-*pyrrol-2-carbonitril wurden in 1.6 ml Formamid vorgelegt und in der Mikrowelle auf 140°C erhitzt. Nach 10 h wurde die Mischung auf RT abgekühlt und mit Wasser und viel Ethylacetat versetzt. Die abgetrennte organische Phase wurde im Vakuum weitgehend eingeengt und die verbleibende Suspension mit Acetonitril behandelt. Der ausgefallene Feststoff wurde abgesaugt und im Hochvakuum getrocknet. Erhalten wurden 456 mg des Zielprodukts (70.7% d. Th.).
LC-MS (Methode 3): Rₜ = 1.77 min; m/z = 317 (M+H)⁺
⁺H-NMR (400 MHz, DMSO-d₆): δ = 8.03 (s, 1H), 7.89 (s, 1H), 7.30-7.16 (m, 7H), 7.03 (d, 2H), 4.95 (br. s, 2H), 3.81 (s, 3H).

### Beispiel 8A

### 5-(4-Methoxyphenyl)-6-phenylpyrrolo[2,1-f][1,2,4]triazin-4(3H)-on

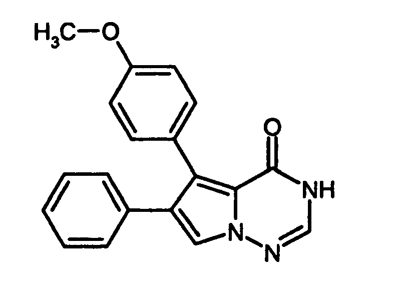

2.4 ml (25.9 mmol) Essigsäureanhydrid wurden auf 0°C gekühlt und tropfenweise mit 1.2 ml (31.1 mmol) Ameisensäure versetzt. Die Mischung wurde 30 min bei 0°C gerührt. Anschließend wurden 300 mg (1.04 mmol) 1-Amino-3-(4-methoxyphenyl)-4-phenyl-1*H-*pyrrol-2-carbonitril zugegeben. Die Reaktionsmischung wurde auf 130°C (Badtemperatur) erhitzt und 24 h gerührt. Nach Abkühlen wurde das Reaktionsgemisch im Hochvakuum eingeengt und der Rückstand in wenig DMSO aufgenommen. Das Produkt wurde durch präparative RP-HPLC (Eluent: Acetonitril/Wasser-Gradient) isoliert. Erhalten wurden 47.8 mg der Zielverbindung (14.5% d. Th.).
LC-MS (Methode 1): Rₜ = 2.30 min; m/z = 318 (M+H)^{+ 1}H-NMR (500 MHz, DMSO-d₆): δ = 11.54 (s, 1H), 7.84 (s, 1H), 7.83 (s, 1H), 7.25 (m, 2H), 7.21-7.15 (m, 4H), 6.85 (d, 2H), 3.78 (s, 3H).

### Beispiel 9A

### 4-Chlor-5-(4-methoxyphenyl)-6-phenylpyrrolo[2,1-f][1,2,4]triazin

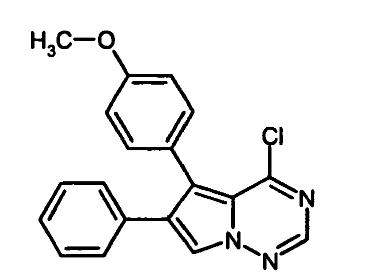

76 mg (0.24 mmol) 5-(4-Methoxyphenyl)-6-phenylpyrrolo[2,1-fl[1,2,4]triazin-4(3*H*)-on wurden bei RT mit 0.45 ml (4.79 mmol) Phosphoroxychlorid versetzt. Die Suspension wurde 3 h unter Rückfluss erhitzt, wobei der Feststoff in Lösung ging. Nach Abkühlen wurde mit Dichlormethan verdünnt und mit Wasser und Ammoniak-Lösung versetzt (pH-Wert der wässrigen Phase ca. 9). Nach Phasentrennung wurde die wässrige Phase zweimal mit Dichlormethan rückextrahiert. Man vereinigte alle organischen Phasen, trocknete über Magnesiumsulfat und engte im Vakuum ein. Nach präparativer RP-HPLC (Eluent: Acetonitril/Wasser-Gradient) wurden 62.7 mg des Zielprodukts erhalten (78.0% d. Th.).
LC-MS (Methode 3): R₁ = 2.43 min; m/z = 336 (M+H)⁺
¹H-NMR (500 MHz, DMSO-d₆): δ = 8.61 (s, 1H), 8.42 (s, 1H), 7.30-7.23 (m, 7H), 6.97 (d, 2H), 3.80 (s, 3H).

### Ausführungsbeispiele:

### Beispiel 1

### (6-{[5-(4-Methoxyphenyl)-6-phenylpyrrolo[2,1-f][1,2,4]triazin-4-yl]amino}hexansäureethylester

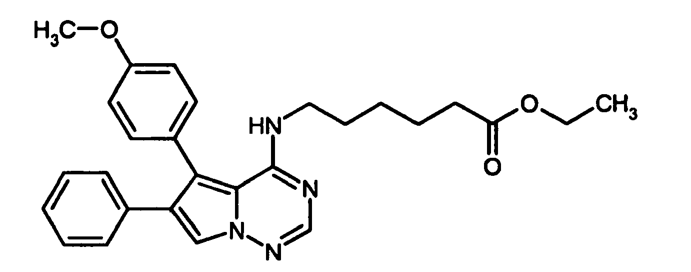

Zu einer Mischung von 87.0 mg (0.275 mmol) 4-Amino-5-(4-methoxyphenyl)-6-phenylpyrrolo-[2,1-f][1,2,4]triazin und 92.0 mg (0.412 mmol) 6-Bromhexansäureethylester in 0.32 ml abs. DMF wurden bei 0°C 11.6 mg (0.289 mol, 60%-ig) Natriumhydrid gegeben. Die Reaktionsmischung wurde langsam auf RT erwärmt und 2 h bei dieser Temperatur gerührt, bevor auf Wasser gegeben wurde. Man extrahierte gründlich mit Ethylacetat, wusch die organische Phase mit ges. Natriumchlorid-Lösung, trocknete über Magnesiumsulfat und engte im Vakuum ein. Das Rohprodukt wurde durch präparative RP-HPLC aufgereinigt (Eluent: Acetonitril/Wasser-Gradient). Erhalten wurden 49.4 mg der Zielverbindung (39.2% d. Th.).
LC-MS (Methode 3): Rₜ = 2.53 min; m/z = 459 (M+H)⁺
¹H-NMR (500 MHz, DMSO-d₄): δ = 7.99 (s, 1H), 7.94 (s, 1H), 7.29 (d, 2H), 7.28-7.17 (m, 5H), 7.03 (d, 2H), 5.28 (t, 1H), 4.03 (q, 2H), 3.82 (s, 3H), 3.34 (q, 2H), 2.24 (t, 2H), 1.49-1.41 (m, 2H), 1.40-1.43 (m, 2H), 1.17 (t, 3H), 1.13-1.05 (m, 2H).

### Beispiel 2

### (6R)-6-{[5-(4-Methoxyphenyl)-6-phenylpyrrolo[2,1-f][1,2,4]triazin-4-yl]oxy}heptansäure-tert.-butylester

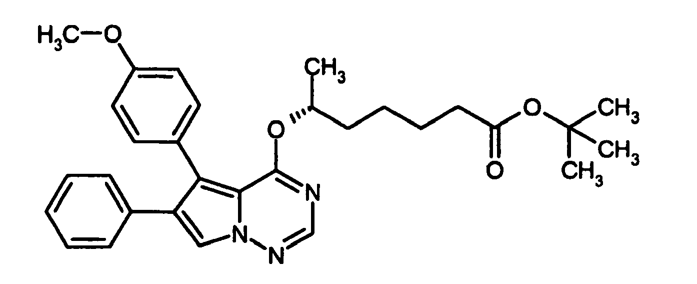

Zu einer Lösung von 61.0 mg (0.182 mmol) 4-Chlor-5-(4-methoxyphenyl)-6-phenylpyrrolo[2,1-f]-[1,2,4]triazin und 51.4 mg (0.254 mmol) (-)-6-Hydroxyheptansäure-*tert*.-butylester in 0.27 ml abs. THF wurden bei -5°C unter Argonatmosphäre 0.24 ml (0.24 mmol) einer 1 M Lösung von P4-Phosphazen-Base in THF getropft. Nach Ende der Zugabe wurde die Mischung auf 0°C erwärmt und 40 min gerührt. Das Reaktionsgemisch wurde danach im Vakuum eingeengt und der Rückstand direkt durch präparative RP-HPLC aufgereinigt (Eluent: Acetonitril/Wasser-Gradient). Erhalten wurden 21.2 mg des Zielprodukts (23.3% d. Th.).
LC-MS (Methode 4): Rₜ = 3.29 min; m/z = 502 (M+H)⁺
¹H-NMR (400 MHz, CDCl₃): δ = 7.99 (s, 1H), 7.81 (s, 1H), 7.30-7.20 (m, 7H), 6.83 (d, 2H), 5.30 (m, 1H), 3.84 (s, 3H), 2.12 (t, 2H), 1.54-1.45 (m, 4H), 1.42 (s, 9H), 1.28 (d, 3H), 1.22-1.10 (m, 2H).

### Allgemeine Vorschrift A: Hydrolyse von Methyl- oder Ethylestern zu den entsprechenden Carbonsäuren

Zu einer Lösung des Methyl- oder Ethylesters in THF oder THF/Methanol (1:1) (Konzentration ca. 0.05 bis 0.5 mol/l) werden bei RT 1.5 bis 10 eq. Natriumhydroxid als 1 N wässrige Lösung gegeben. Die Mischung wird für einen Zeitraum von 0.5-18 h bei RT gerührt und dann mit 1 N Salzsäure neutralisiert oder schwach angesäuert. Falls dabei ein Feststoff ausfällt, kann das Produkt durch Filtration, Waschen mit Wasser und Trocknen im Hochvakuum isoliert werden. Alternativ wird die Zielverbindung direkt aus dem Rohprodukt, gegebenenfalls nach extraktiver Aufarbeitung mit Dichlormethan, durch präparative RP-HPLC isoliert (Eluent: Acetonitril/Wasser-Gradient) oder durch Verrühren in einem inerten Lösungsmittel gereinigt.

Das folgende Ausführungsbeispiel wurde gemäß der Allgemeinen Vorschrift A erhalten:

| **Beispiel** | **Struktur** | **Analytische Daten** |
|---|---|---|
| **3** | 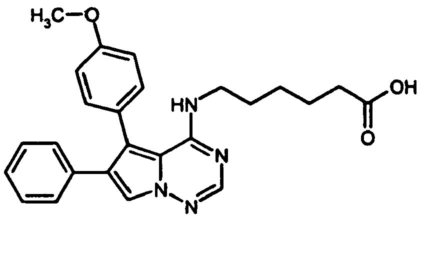 | LC-MS (Methode 1): R₁ = 2.49 min; m/z = 431 (M+H)¹ 1H-NMR (400 MHz, DMSO-d₆): δ = 12.02 (s, 1H), 8.01 (s, 1H), 7.95 (s, 1H), 7.31 (d, 2H), 7.28-7.16 (m, 5H), 7.05 (d, 2H), 5.29 (t, 1H), 3.84 (s, 3H), 3.40-3.35 (m, 2H), 2.17 (t, 2H), 1.47-1.32 (m, 4H), 1.14-1.05 (m, 2H). |

### Allgemeine Vorschrift B: Spaltung von tert.-Butylestern zu den entsprechenden Carbonsäuren

Zu einer Lösung des *tert*.-Butylesters in Dichlormethan (Konzentration 0.1 bis 1.0 mol/l; zusätzlich optional ein Tropfen Wasser) wird bei 0°C bis RT tropfenweise Trifluoressigsäure (TFA) hinzugefügt, bis ein Verhältnis Dichlormethan/TFA von ca. 2:1 bis 1:2 erreicht ist. Die Mischung wird 1-18 h bei RT gerührt und dann im Vakuum eingeengt. Alternativ wird das Reaktionsgemisch mit Dichlormethan verdünnt, mit Wasser und ges. Natriumchlorid-Lösung gewaschen, getrocknet und im Vakuum eingeengt. Das Rohprodukt kann, falls erforderlich, beispielsweise durch präparative RP-HPLC gereinigt werden (Eluent: Acetonitril/Wasser-Gradient).

Das folgende Ausführungsbeispiel wurde gemäß der Allgemeinen Vorschrift B erhalten:

| **Beispiel** | **Struktur** | **Analytische Daten** |
|---|---|---|
| **4** | 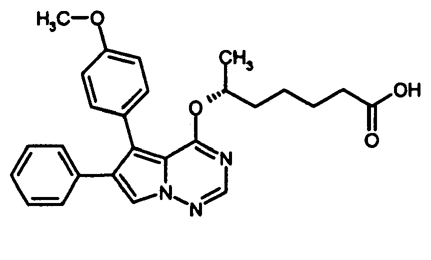 | LC-MS (Methode 4): Rₜ = 2.66 min; m/z = 446 (M+H)⁺ ¹H-NMR (500 MHz, DMSO-d₆): δ = 11.94 (br. s, 1H), 8.20 (s, 1H), 8.14 (s, 1H), 7.29-7.14 (m, 7H), 6.89 (d, 2H), 5.27 (m, 1H), 3.78 (s, 3H), 2.10 (t, 2H), 1.49-1.32 (m, 4H), 1.21 (d, 3H), 1.15-1.02 (m, 2H). [α]_{D}²⁰ = -81°, c = 0.105, Chloroform. |

### B. Bewertung der pharmakologischen Wirksamkeit

Die pharmakologische Wirkung der erfindungsgemäßen Verbindungen kann in folgenden Assays gezeigt werden:

### B-1. Bindungsstudien mit Prostacyclin-Rezeptoren (IP-Rezeptoren) von humanen Thrombozytenmembranen

Zur Gewinnung von Thrombozytenmembranen werden 50 ml Humanblut (Buffy coats mit CDP-Stabilizer, Fa. Maco Pharma, Langen) für 20 min bei 160 x g zentrifugiert. Der Überstand (plättchenreiches Plasma, PRP) wird abgenommen und anschließend nochmals bei 2000 x g für 10 min bei Raumtemperatur zentrifugiert. Das Sediment wird in 50 mM Tris-(hydroxymethyl)-aminomethan, welches mit 1 N Salzsäure auf einen pH-Wert von 7.4 eingestellt ist, re-suspendiert und bei -20°C über Nacht aufbewahrt. Am folgenden Tag wird die Suspension bei 80000 x g und 4°C 30 min lang zentrifugiert. Der Überstand wird verworfen. Das Sediment wird in 50 mM Tris-(hydroxymethyl)-aminomethan/Salzsäure, 0.25 mM Ethylendiamintetraessigsäure (EDTA), pH 7.4 re-suspendiert und danach nochmals bei 80000 x g und 4°C für 30 min zentrifugiert. Das Membransediment wird in Bindungspuffer (50 mM Tris-(hydroxymethyl)-aminomethan/Salzsäure, 5 mM Magnesiumchlorid, pH 7.4) aufgenommen und bis zum Bindungsversuch bei -70°C gelagert.

Für den Bindungsversuch werden 3 nM ³H-Iloprost (592 GBq/mmol, Fa. AmershamBioscience) 60 min lang mit 300-1000 µg/ml humanen Thrombozytenmembranen pro Ansatz (max. 0.2 ml) in Gegenwart der Testsubstanzen bei Raumtemperatur inkubiert. Nach dem Abstoppen werden die Membranen mit kaltem Bindungspuffer versetzt und mit 0.1% Rinderserumalbumin gewaschen. Nach Zugabe von Ultima Gold-Szintillator wird die an den Membranen gebundene Radioaktivität mittels eines Szintillationszählers quantifiziert. Die nicht-spezifische Bindung wird als Radioaktivität in Gegenwart von 1 µM Iloprost (Fa. Cayman Chemical, Ann Arbor) definiert und beträgt in der Regel < 25% der gebundenen Gesamt-Radioaktivität. Die Bindungsdaten (IC₅₀-Werte) werden mittels des Programmes GraphPad Prism Version 3.02 bestimmt.

Repräsentative Ergebnisse zu den erfindungsgemäßen Verbindungen sind in Tabelle 1 aufgeführt:

**Tabelle 1**

| **Beispiel Nr.** | **IC₅₀ [nM]** |
|---|---|
| 3 | 636 |
| 4 | 224 |

### B-2. IP-Rezeptor-Stimulierung auf Ganzzellen

Die IP-agonistische Wirkung von Testsubstanzen wird mit Hilfe der humanen Erythroleukämie-Zelllinie (HEL), die den IP-Rezeptor endogen exprimiert, bestimmt [Murray, R., FEBS Letters 1989, 1: 172-174]. Dazu werden die Suspensionszellen (4 x 10⁷ Zellen/ml) in Puffer [10 mM HEPES (4-(2-Hydroxyethyl)-1-piperazinethansulfonsäure) / PBS (Phosphat-gepufferte Salzlösung, Fa. Oxoid, UK)], 1 mM Calciumchlorid, 1 mM Magnesiumchlorid, 1 mM IBMX (3-Isobutyl-1-methylxanthin), pH 7.4, mit der jeweiligen Testsubstanz 5 Minuten lang bei 30°C inkubiert. Anschließend wird die Reaktion durch Zugabe von 4°C kaltem Ethanol gestoppt und die Ansätze weitere 30 Minuten bei 4°C gelagert. Danach werden die Proben bei 10000 x g und 4°C zentrifugiert. Der resultierende Überstand wird verworfen und das Sediment zur Bestimmung der Konzentration an cyclischem Adenosinmonophosphat (cAMP) in einem kommerziell erhältlichen cAMP-Radioimmunoassay (Fa. IBL, Hamburg) eingesetzt. IP-Agonisten führen in diesem Test zu einem Anstieg der cAMP-Konzentration, IP-Antagonisten sind wirkunglos. Die effektive Konzentration (EC₅₀-Werte) wird mittels des Programmes GraphPad Prism Version 3.02 bestimmt.

### B-3. Thrombozytenaggregationshemmung in vitro

Zur Bestimmung der Thrombozytenaggregationshemmung wird Blut von gesunden Probanden beiderlei Geschlechts verwendet. Einem Teil 3.8%-iger Natriumcitrat-Lösung als Koagulans werden 9 Teile Blut zugemischt. Das Blut wird mit 900 U/min für 20 min zentrifugiert. Der pH-Wert des gewonnenen plättchenreichen Plasmas wird mit ACD-Lösung (Natriumcitrat/Citronensäure/Glucose) auf pH 6.5 eingestellt. Die Thrombozyten werden anschließend abzentrifugiert, in Puffer aufgenommen und erneut abzentrifugiert. Der Thrombozyten-Niederschlag wird in Puffer aufgenommen und zusätzlich mit 2 mmol/l Calciumchlorid re-suspendiert.

Für die Aggregationsmessungen werden Aliquots der Thrombozytensuspension mit der Prüfsubstanz 10 min lang bei 37°C inkubiert. Anschließend wird die Aggregation durch Zugabe von ADP induziert und mittels der turbidometrischen Methode nach Born im Aggregometer bei 37°C bestimmt [Born G.V.R., J. Physiol. (London) 168, 178-179 (1963)].

### B-4. Blutdruckmessung an narkotisierten Ratten

Männliche Wistar-Ratten mit einem Körpergewicht von 300-350 g werden mit Thiopental (100 mg/kg i.p.) anästhesiert. Nach der Tracheotomie wird die Arteria femoralis zur Blutdruckmessung katheterisiert. Die zu prüfenden Substanzen werden als Lösung oral mittels Schlundsonde oder über die Femoralvene intravenös in einem geeigneten Vehikel verabreicht.

### B-5. PAH-Modell im narkotisierten Hund

Bei diesem Tiermodell der pulmonalen arteriellen Hypertonie (PAH) werden Mongrel-Hunde mit einem Körpergewicht von ca. 25 kg eingesetzt. Die Narkose wird eingeleitet durch langsame i.v.-Gabe von 25 mg/kg Natrium-Thiopental (Trapanal^{®}) und 0.15 mg/kg Alcuroniumchlorid (Alloferin^{®}) und während des Experimentes aufrecht erhalten mittels einer Dauerinfusion von 0.04 mg/kg/h Fentanyl^{®}, 0.25 mg/kg/h Droperidol (Dehydrobenzperidol^{®}) und 15 µg/kg/h Alcuroniumchlorid (Alloferin^{®}). Reflektorische Einflüsse auf die Herzfrequenz durch Blutdrucksenkung werden durch autonome Blockade [Dauerinfusion von Atropin (ca. 10 µg/kg/h) und Propranolol (ca. 20 µg/kg/h)] minimiert. Nach der Intubation werden die Tiere über eine Beatmungsmaschine mit konstantem Atemvolumen beatmet, so dass eine endtidale CO₂-Konzentration von etwa 5% erreicht wird. Die Beatmung erfolgt mit Raumluft, angereichert mit ca. 30% Sauerstoff (Normoxie). Zur Messung der hämodynamischen Parameter wird ein mit Flüssigkeit gefüllter Katheter in die *A. femoralis* zur Messung des Blutdrucks implantiert. Ein zweilumiger Swan-Ganz^{®}-Katheter wird über die *V. jugularis* in die Pulmonalarterie eingeschwemmt (distales Lumen zur Messung des pulmonal-arteriellen Drucks, proximales Lumen zur Messung des zentralen Venendrucks). Der linksventrikuläre Druck wird nach Einführung eines Mikro-Tip-Katheters (Millar^{®} Instruments) über die *A. carotis* in den linken Ventrikel gemessen und davon der dP/dt-Wert als Maß für die Kontraktilität abgeleitet. Substanzen werden i.v. über die *V. femoralis* appliziert. Die hämodynamischen Signale werden mittels Druckaufnehmern/Verstärkern und PONEMAH^{®} als Datenerfassungssoftware aufgezeichnet und ausgewertet.

Um eine akute pulmonale Hypertonie zu induzieren, wird als Stimulus entweder Hypoxie oder eine kontinuierliche Infusion von Thromboxan A₂ oder einem Thromboxan A₂-Analogon eingesetzt. Akute Hypoxie wird induziert durch graduierte Erniedrigung des Sauerstoffs in der Beatmungsluft auf ca. 14%, so dass der mPAP auf Werte von >25 mm Hg ansteigt. Bei einem Thromboxan A₂-Analogon als Stimulus werden 0.21-0.32 µg/kg/min U-46619 [9,11-Dideoxy-9α,11α-epoxy-methano-prostaglandin F_{2α} (Fa. Sigma)] infundiert, um den mPAP auf >25 mm Hg zu erhöhen.

### B-6. PAH-Modell im narkotisierten Göttinger Minipig

Bei diesem Tiermodell der pulmonalen arteriellen Hypertonie (PAH) werden Göttinger Minischweine mit einem Körpergewicht von ca. 25 kg eingesetzt. Die Narkose wird eingeleitet durch 30 mg/kg Ketamin (Ketavet^{®}) i.m., gefolgt von einer i.v.-Gabe von 10 mg/kg Natrium-Thiopental (Trapanal^{®}); sie wird während des Experiments aufrecht erhalten mittels Inhalationsnarkose aus Enfluran (2-2.5%) in einer Mischung aus Raumluft, angereichert mit ca. 30-35% Sauerstoff / N₂O (1:1.5). Zur Messung der hämodynamischen Parameter wird ein mit Flüssigkeit gefüllter Katheter in die *A. carotis* zur Messung des Blutdrucks implantiert. Ein zweilumiger Swan-Ganz^{®}-Katheter wird über die *V. jugularis* in die Pulmonalarterie eingeschwemmt (distales Lumen zur Messung des pulmonal-arteriellen Drucks, proximales Lumen zur Messung des zentralen Venendrucks). Der linksventrikuläre Druck wird nach Einführung eines Mikro-Tip-Katheters (Millar^{®} Instruments) über die *A. carotis* in den linken Ventrikel gemessen und davon der dP/dt-Wert als Maß für die Kontraktilität abgeleitet. Substanzen werden i.v. über die Femoralvene appliziert. Die hämodynamischen Signale werden mittels Druckaufnehmern/Verstärkern und PONEMAH^{®} als Datenerfassungssoftware aufgezeichnet und ausgewertet.

Um eine akute pulmonale Hypertonie zu induzieren, wird als Stimulus eine kontinuierliche Infusion eines Thromboxan A₂-Analogons eingesetzt. Hierbei werden 0.12-0.14 µg/kg/min U-46619 [9,11-Dideoxy-9α,11α-epoxymethano-prostaglandin F_{2α} (Fa. Sigma)] infundiert, um den mPAP auf >25 mm Hg zu erhöhen.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Z

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 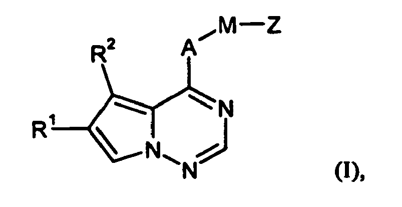 in welcher
A für O oder N-R³ steht, worin
R³ Wasserstoff, (C₁-C₆)-Alky1, (C₃-C₇)-Cycloalkyl oder (C₄-C₇)-Cycloalkenyl bedeutet,
M für eine Gruppe der Formel 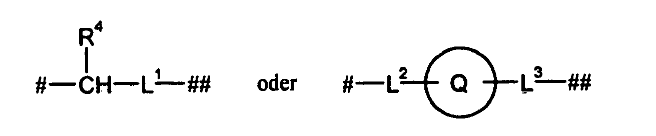 steht, worin
# die Verknüpfungstelle mit der Gruppe A und
## die Verknüpfungsstelle mit der Gruppe Z bedeuten,
R⁴ Wasserstoff oder (C₁-C₄)-Alkyl, welches mit Hydroxy oder Amino sub- stituiert sein kann, bedeutet,
L¹ (C₁-C₇)-Alkandiyl oder (C₂-C₇-Alkendiyl, welche ein- oder zweifach mit Fluor substituiert sein können, oder eine Gruppe der Formel *-L^{1A}-V-L^{1B}** bedeutet, worin
* die Verknüpfungsstelle mit der Gruppe -CHR⁴,
** die Verknüpfungsstelle mit der Gruppe Z,
L^{1A} (C₁-C₅)-Alkandiyl, welches ein- oder zweifach, gleich oder ver- schieden, mit (C₁-C₄)-Alkyl und/oder (C₁-C₄)-Alkoxy substituiert sein kann,
L^{1B} eine Bindung oder (C₁-C₃)-Alkandiyl, welches ein- oder zweifach mit Fluor substituiert sein kann,
und
V O oder N-R⁵, worin
R⁵ Wasserstoff, (C₁-C₆)-Alkyl oder (C₃-C₇)-Cycloalkyl be- deutet,
darstellen,
L² eine Bindung oder (C₁-C₄)-Alkandiyl bedeutet,
L³ (C₁-C₄)-Alkandiyl bedeutet, welches ein- oder zweifach mit Fluor substi- tuiert sein kann und in welchem eine Methylengruppe gegen O oder N-R⁶ ausgetauscht sein kann, worin
R⁶ Wasserstoff, (C₁-C₆)-Alkyl oder (C₃-C₇-Cycloalkyl darstellt, oder (C₂-C₄)-Alkendiyl bedeutet,
und
Q (C₃-C₇)-Cycloalkyl, (C₄-C₇)-Cycloalkenyl, Phenyl, 5- bis 7-gliedriges Heterocyclyl oder 5- oder 6-gliedriges Heteroaryl bedeutet, welche jeweils bis zu zweifach, gleich oder verschieden, mit Resten ausgewählt aus der Reihe Fluor, Chlor, (C₁-C₄)-Alkyl, Trifluormethyl, Hydroxy, (C₁-C₄)- Alkoxy, Trifluormethoxy, Amino, Mono-(C₁-C₄)-alkylamino und Di-(C₁- C₄)-alkylamino substituiert sein können, wobei (C₁-C₄)-Alkyl seinerseits mit Hydroxy, (C₁-C₄)-Alkoxy, Amino, Mono- oder Di-(C₁-C₄)-alkylamino substituiert sein kann,
Z für eine Gruppe der Formel 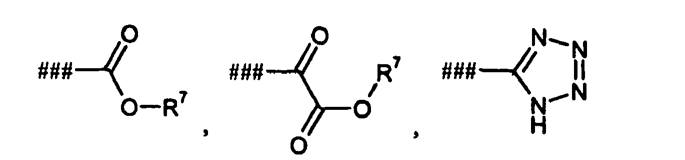 oder 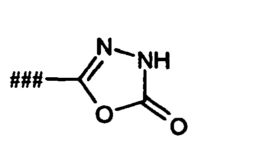 steht, worin
### die Verknüpfungsstelle mit der Gruppe L¹ beziehungsweise L³
und
R⁷ Wasserstoff oder (C₁-C₄)-Alkyl bedeutet,
und
R¹ und R² gleich oder verschieden sind und unabhängig voneinander für (C₃-C₇)-Cycloalkyl, (C₄-C₇)-Cycloalkenyl, Phenyl, 5- bis 7-gliedriges Heterocyclyl oder 5- oder 6-gliedriges Heteroaryl stehen, welche jeweils ein- bis dreifach, gleich oder verschieden, mit Resten ausgewählt aus der Reihe Halogen, Cyano, Nitro, (C₁-C₆)-Alkyl, (C₂-C₆Alkenyl, 1(C₂-C₄)-Alkinyl, (C₃-C₇)-Cycloalkyl, (C₄-C₇)-Cycloalkenyl, (C₁-C₆)-Alkoxy, Trifluormethyl, Trifluormethoxy, (C₁-C₆-Alkylthio, (C₁-C₆)-Acyl, Amino, Mono-(C₁-C₆)-alkylamino, Di-C₁-C₆-alkylamino und (C₁-C₆-Acylamino substituiert sein können,
wobei (C₁-C₆)-Alkyl und (C₁-C₆)-Alkoxy ihrerseits jeweils mit Cyano, Hydroxy, (C₁-C₄)-Alkoxy, (C₁-C₄)-Alkylthio, Amino, Mono- oder Di-(C₁-C₄)-alkylamino substituiert sein können,
oder
R¹ und/oder R² für Phenyl stehen, worin zwei an benachbarte Ring-Kohlenstoffatome gebundene Reste zusammen eine Gruppe der Formel -O-CH₂O-, -O-CHF-O-, -O-CF₂-O-, -O-CH₂-CH₂-O- oder -O-CF₂-CF₂-O- bilden,
sowie ihre Salze, Solvate und Solvate der Salze.

2. Verbindung der Formel (I) nach Anspruch 1, in welcher
A für O oder NH steht,
M für eine Gruppe der Formel 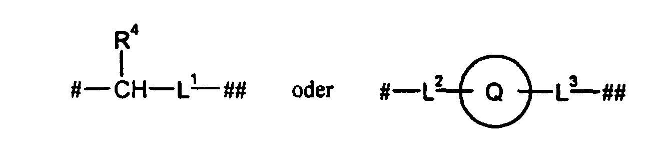 steht, worin
# die Verknüpfungstelle mit der Gruppe A und
## die Verknüpfungsstelle mit der Gruppe Z bedeuten,
R⁴ Wasserstoff, Methyl oder Ethyl bedeutet,
L¹ (C₃-C₇)-Alkandiyl, (C₃-C₇)-Alkendiyl oder eine Gruppe der Formel *-L^{1A}-V-L^{1B}-** bedeutet, worin
* die Verknüpfungsstelle mit der Gruppe -CHR⁴,
** die Verknüpfungsstelle mit der Gruppe Z,
L^{1A} (C₁-C₃)-Alkandiyl, welches ein- oder zweifach mit Methyl substi- tuiert sein kann,
L^{1B} (C₁-C₃)-Alkandiyl und V O oder N-CH₃ darstellen,
L² eine Bindung, Methylen, Ethan-1,1-diyl oder Ethan-1,2-diyl bedeutet,
L³ (C₁-C₃)-Alkandiyl oder eine Gruppe der Formel •-W-CH₂-•• oder •-W-CH₂-CH₂-•• bedeutet, worin
• die Verknüpfungsstelle mit dem Ring Q,
••die Verknüpfungsstelle mit der Gruppe Z
und
W O oder N-R⁶, worin
R⁶ Wasserstoff oder (C₁-C₃)-Alkyl bedeutet, darstellen,
und
Q Cyclobutyl, Cyclopentyl, Cyclopentenyl, Cyclohexyl, Pyrrolidinyl, Piperi- dinyl, Tetrahydrofuranyl, Tetrahydropyranyl, Morpholinyl oder Phenyl bedeutet, welche jeweils bis zu zweifach, gleich oder verschieden, mit Resten ausgewählt aus der Reihe Fluor, Methyl, Ethyl, Trifluomethyl, Hydroxy, Methoxy und Ethoxy substituiert sein können,
Z für eine Gruppe der Formel 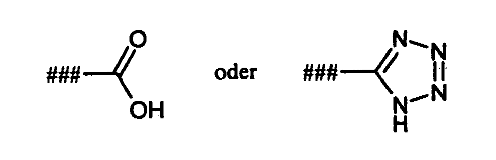 steht, worin
### die Verknüpfungsstelle mit der Gruppe L¹ beziehungsweise L³ bedeutet,
und
R¹ und R² gleich oder verschieden sind und unabhängig voneinander für Cyclopenten-1-yl, Cyclohexen-1-yl, Phenyl, Thienyl oder Pyridyl stehen, welche jeweils ein- oder zweifach, gleich oder verschieden, mit Resten ausgewählt aus der Reihe Fluor, Chlor, Cyano, (C₁-C₄-Alkyl, (C₂-C₄-Alkenyl, (C₁-C₄)-Alkoxy, Trifluormethyl und Trifluormethoxy substituiert sein können,
sowie ihre Salze, Solvate und Solvate der Salze.

3. Verbindung der Formel (I) nach Anspruch 1 oder 2, in welcher
A für O oder NH steht,
M für die Gruppe der Formel 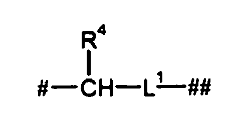 steht, worin
# die Verknüpfungstelle mit der Gruppe A
und
## die Verknüpfungsstelle mit der Gruppe Z bedeuten,
R⁴ Wasserstoff oder Methyl bedeutet,
und
L¹ Butan-1,4-diyl, Pentan-1,5-diyl oder eine Gruppe der Formel *-L^{1A}-O-L^{1B}-** bedeutet, worin
* die Verknüpfungsstelle mit der Gruppe -CHR⁴,
** die Verknüpfungsstelle mit der Gruppe Z,
L^{1A} Methylen oder Ethan-1,2-diyl, welche ein- oder zweifach mit Methyl substituiert sein können,
und
L^{1B}Methylen oder Ethan-1,2-diyl darstellen,
Z für die Gruppe der Formel 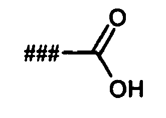 steht, worin
### die Verknüpfungsstelle mit der Gruppe L¹ bedeutet,
R¹ für Phenyl steht, das mit Fluor oder Chlor substituiert sein kann,
und
R² für Phenyl steht, das mit Methyl, Ethyl, Methoxy oder Ethoxy substituiert sein kann,
sowie ihre Salze, Solvate und Solvate der Salze.

4. Verfahren zur Herstellung von Verbindungen, wie in den Ansprüchen 1 bis 3 definiert, in welchen Z für -COOH oder -C(=O)-COOH steht, **dadurch gekennzeichnet, dass** man entweder
[A] Verbindungen der Formel (II) 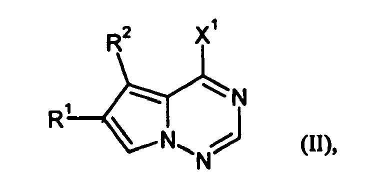 in welcher R¹ und R² die in den Ansprüchen 1 bis 3 angegebenen Bedeutungen haben
und
X¹ für eine Abgangsgruppe wie beispielsweise Halogen, insbesondere für Chlor steht,
in einem inerten Lösungsmittel in Gegenwart einer Base mit einer Verbindung der Formel (III) 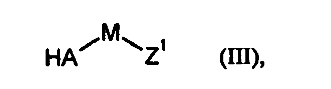 in welcher A und M die in den Ansprüchen 1 bis 3 angegebenen Bedeutungen haben
und
Z¹ für Cyano oder eine Gruppe der Formel -[C(O)]_{y}-COOR^{7A} steht, worin y die Zahl 0 oder 1 und R^{7A} (C₁-C₄)-Alkyl bedeutet,
zu Verbindungen der Formel (IV) 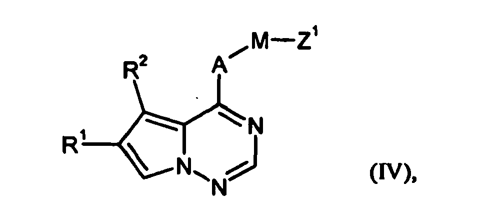 in welcher A, M, Z', R¹ und R² jeweils die oben angegebenen Bedeutungen haben,
oder
[B] Verbindungen der Formel (V) 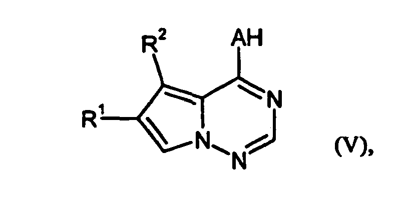 in welcher A, R¹ und R² jeweils die in den Ansprüchen 1 bis 3 angegebenen Bedeutungen haben,
in einem inerten Lösungsmittel in Gegenwart einer Base mit einer Verbindung der Formel (VI) 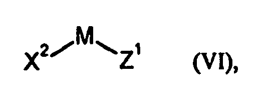 in welcher M die in den Ansprüchen 1 bis 3 und Z¹ die oben angegebene Bedeutung hat
und
x² für eine Abgangsgruppe wie beispielsweise Halogen, Mesylat, Tosylat oder Triflat steht,
zu Verbindungen der Formel (IV) 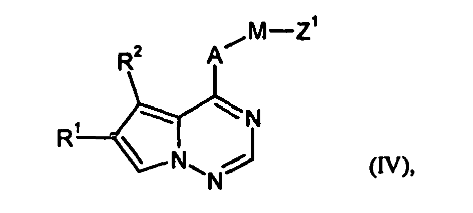 in welcher A, M, Z¹, R¹ und R² jeweils die oben angegebenen Bedeutungen haben, umsetzt
und die Verbindungen der Formel (IV) dann durch Hydrolyse der Ester- bzw. Cyano-Gruppe Z¹ in die Carbonsäuren der Formel (I-A) 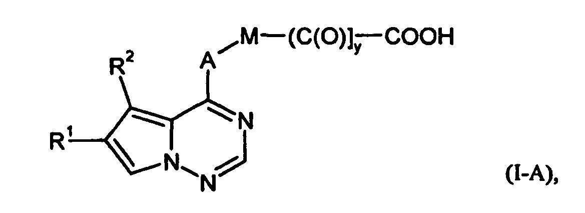 in welcher A, M, R¹, R² und y jeweils die oben angegebenen Bedeutungen haben,
überführt und diese gegebenenfalls mit den entsprechenden (i) Lösungsmitteln und/oder (ii) Basen oder Säuren zu ihren Solvaten, Salzen und/oder Solvaten der Salze umsetzt.

5. Verbindung, wie in einem der Ansprüche 1 bis 3 definiert, zur Behandlung und/oder Prophylaxe von Krankheiten.

6. Verwendung einer Verbindung, wie in einem der Ansprüche 1 bis 3 definiert, zur Herstellung eines Arzneimittels zur Behandlung und/oder Prophylaxe von Angina pectoris, pulmonaler Hypertonie, thromboembolischen Erkrankungen und peripheren Verschlusskrankheiten.

7. Arzneimittel enthaltend eine Verbindung, wie in einem der Ansprüche 1 bis 3 definiert, in Kombination mit einem inerten, nicht-toxischen, pharmazeutisch geeigneten Hilfsstoff.

8. Arzneimittel enthaltend eine Verbindung, wie in einem der Ansprüche 1 bis 3 definiert, in Kombination mit einem weiteren Wirkstoff.

9. Arzneimittel nach Anspruch 7 oder 8 zur Behandlung und/oder Prophylaxe von Angina pectoris, pulmonaler Hypertonie, thromboembolischen Erkrankungen und peripheren Verschlusskrankheiten.

## Claims

1. Compound of the formula (I) 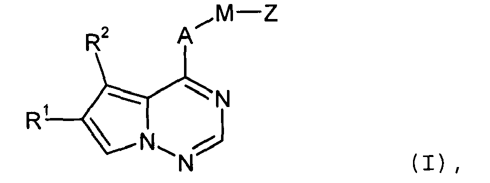 in which
A represents O or N-R³ in which
R³ represents hydrogen, (C₁-C₆) -alkyl, (C₃-C₇)-cycloalkyl or (C₄-C₇) -cycloalkenyl,
M represents a group of the formula 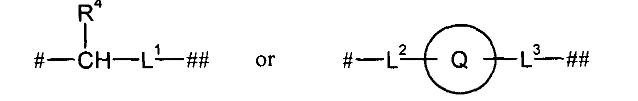 in which
# represents the point of attachment to the group A
and
## represents the point of attachment to the group Z,
R⁴ represents hydrogen or (C₁-C₄)-alkyl which may be substituted by hydroxyl or amino,
L¹ represents (C₁-C₇)-alkanediyl or (C₂-C₇) - alkenediyl which may be mono- or disubstituted by fluorine, or a group of the formula *-L^{1A}-V-L^{1B}-** in which * represents the point of attachment to the group -CHR⁴, ** represents the point of attachment to the group Z, L^{1A} represents (C₁-C₅) -alkanediyl which may be mono- or disubstituted by identical or different radicals from the group consisting of (C₁- C₄)-alkyl and (C₁-C₄) -alkoxy, L^{1B} represents a bond or ( C₁-C₃) - alkanediyl which may be mono- or disubstituted by fluorine, and V represents O or N-R⁵ in which R⁵ represents hydrogen, (C₁-C₆) - alkyl or (C₃-C₇) -cycloalkyl,
L² represents a bond or (C₁-C₄) -alkanediyl,
L³ represents (C₁-C₄) -alkanediyl which may be mono- or disubstituted by fluorine and in which a methylene group may be replaced by O or N-R⁶ in which R⁶ represents hydrogen, (C₁-C₆) -alkyl or (C₃-C₇) -cycloalkyl, or represents (C₂-C₄) -alkenediyl,
and
Q represents (C₃-C₇) -cycloalkyl, (C₄-C₇) - cycloalkenyl, phenyl, 5- to 7-membered heterocyclyl or 5- or 6-membered heteroaryl, each of which may be substituted up to two times by identical or different radicals from the group consisting of fluorine, chlorine, (C₁- C₄)-alkyl, trifluoromethyl, hydroxyl, (C₁-C₄)-alkoxy, trifluoromethoxy, amino, mono- (C₁-C₄) -alkylamino and di- (C₁-C₄) - alkylamino, where (C₁-C₄) -alkyl for its part may be substituted by hydroxyl, (C₁-C₄)-alkoxy, amino, mono- or di- (C₁-C₄) -alkylamino,
Z represents a group of the formula 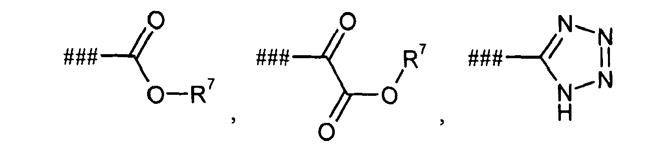 or 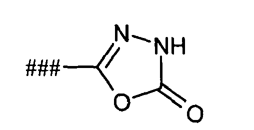 in which
### represents the point of attachment to the group L¹ or L³
and
R⁷ represents hydrogen or (C₁-C₄) -alkyl,
and
R¹ and R² are identical or different and independently of one another represent (C₃- C₇)-cycloalkyl, (C₄-C₇)-cycloalkenyl, phenyl, 5- to 7-membered heterocyclyl or 5- or 6- membered heteroaryl, each of which may be mono- to trisubstituted by identical or different radicals from the group consisting of halogen, cyano, nitro, (C₁-C₆) -alkyl, (C₂- C₆)-alkenyl, (C₂-C₄)-alkynyl, (C₃-C₇)- cycloalkyl, (C₄-C₇)-cycloalkenyl, (C₁-C₆) - alkoxy, trifluoromethyl, trifluoromethoxy, (C₁-C₆) -alkylthio, (C₁-C₆) -acyl, amino, mono- (C₁-C₆) -alkylamino, di- (C₁-C₆) -alkylamino and (C₁-C₆) -acylamino, where (C₁-C₆) -alkyl and (C₁-C₆) -alkoxy for their part may in each case be substituted by cyano, hydroxyl, (C₁-C₄) -alkoxy, (C₁-C₄) - alkylthio, amino, mono- or di-(C₁-C₄)- alkylamino,
or
R¹ and/or R² represent(s) phenyl in which two radicals attached to adjacent ring carbon atoms together form a group of the formula -O-CH₂-O-, -O-CHF-O-, -O-CF₂-O-, -O-CH₂-CH₂-O- or -O-CF₂-CF₂-O-,
and also its salts, solvates and solvates of the salts.

2. Compound of the formula (I) according to Claim 1 in which
A represents O or NH,
M represents a group of the formula 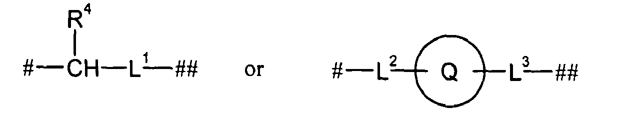 in which
# represents the point of attachment to the group A
and
## represents the point of attachment to the group Z,
R⁴ represents hydrogen, methyl or ethyl,
L¹ represents (C₃-C₇) -alkanediyl, (C₃-C₇)- alkenediyl or a group of the formula *-L^{1A}-V-L^{1B}-** in which * represents the point of attachment to the group -CHR⁴, ** represents the point of attachment to the group Z, L^{1A} represents (C₁-C₃) -alkanediyl which may be mono- or disubstituted by methyl, L^{1B} represents (C₁-C₃) -alkanediyl and V represents O or N-CH₃,
L² represents a bond, methylene, ethane- 1,1-diyl or ethane-1,2-diyl,
L³ represents (C₁-C₃)-alkanediyl or a group of the formula •-W-CH₂-•• or •-W-CH₂-CH₂-•• in which •represents the point of attachment to the ring Q, •• represents the point of attachment to the group Z and w represents O or N-R⁶ in which R⁶ represents hydrogen or (C₁-C₃) - alkyl,
and
Q represents cyclobutyl, cyclopentyl, cyclopentenyl, cyclohexyl, pyrrolidinyl, piperidinyl, tetrahydrofuranyl, tetrahydropyranyl, morpholinyl or phenyl, each of which may be substituted up to two times by identical or different radicals from the group consisting of fluorine, methyl, ethyl, trifluoromethyl, hydroxyl, methoxy and ethoxy,
Z represents a group of the formula 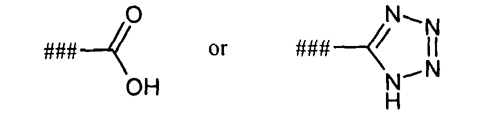 in which
### represents the point of attachment to the group L¹ or L³,
and
R¹ and R² are identical or different and independently of one another represent cyclopenten-1-yl, cyclohexen-1-yl, phenyl, thienyl or pyridyl, each of which may be mono- or disubstituted by identical or different radicals from the group consisting of fluorine, chlorine, cyano, (C₁-C₄) -alkyl, (C₂-C₄) -alkenyl, (C₁-C₄) -alkoxy, trifluoromethyl and trifluoromethoxy,
and also its salts, solvates and solvates of the salts.

3. Compound of the formula (I) according to Claim 1 or 2 in which
A represents O or NH,
M represents the group of the formula 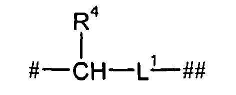 in which
# represents the point of attachment to the group A
and
## represents the point of attachment to the group Z,
R⁴ represents hydrogen or methyl,
and
L¹ represents butane-1,4-diyl, pentane-1,5- diyl or a group of the formula *-L^{1A}-O-L^{1B}-** in which
* represents the point of attachment to the group -CHR⁴,
** represents the point of attachment to the group Z,
L^{1A} represents methylene or ethane-1,2- diyl which may be mono- or disubstituted by methyl, and
L^{1B} represents methylene or ethane-1,2- diyl,
Z represents the group of the formula 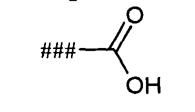 in which
### represents the point of attachment to the group L¹,
R¹ represents phenyl which may be substituted by fluorine or chlorine,
and
R² represents phenyl which may be substituted by methyl, ethyl, methoxy or ethoxy,
and also its salts, solvates and solvates of the salts.

4. Process for preparing compounds as defined in Claims 1 to 3 in which Z represents -COOH or - C(=O)-COOH, **characterized in that** either
[A] compounds of the formula (II) 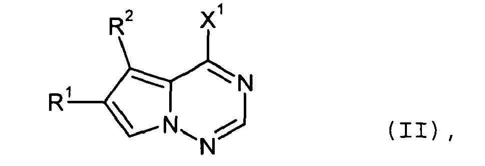 in which R¹ and R² have the meanings given in Claims 1 to 3
and
X¹ represents a leaving group such as, for example, halogen, in particular chlorine,
are reacted in an inert solvent in the presence of a base with a compound of the formula (III) 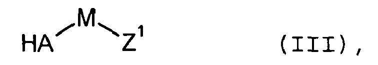 in which A and M have the meanings given in Claims 1 to 3
and
Z¹ represents cyano or a group of the formula - [C(O)]y-COOR^{7A} in which y represents the number 0 or 1 and R^{7A} represents (C₁-C₄) -alkyl,
to give compounds of the formula (IV) 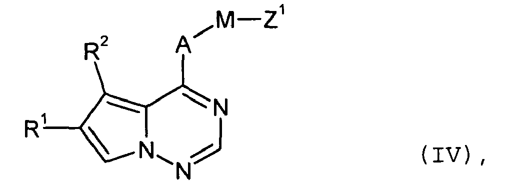 in which A, M, Z¹, R¹ and R² each have the meanings given above,
or
[B] compounds of the formula (V) 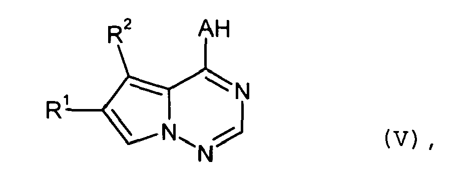 in which A, R¹ and R² each have the meanings given in Claims 1 to 3,
are reacted in an inert solvent in the presence of a base with a compound of the formula (VI) 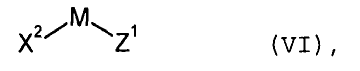 in which M has the meaning given in Claims 1 to 3 and Z¹ has the meaning given above
and
X² represents a leaving group such as, for example, halogen, mesylate, tosylate or triflate,
to give compounds of the formula (IV) 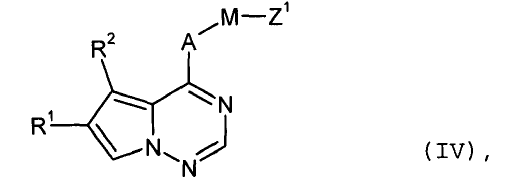 in which A, M, Z¹, R¹ and R² each have the meanings given above,
and the compounds of the formula (IV) are then converted by hydrolysis of the ester or cyano group Z¹ into the carboxylic acids of the formula (I-A) 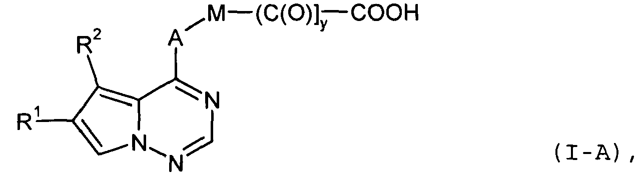 in which A, M, R¹, R² and y each have the meanings given above,
and these are, if appropriate, converted into their solvates, salts and/or solvates of the salts using the appropriate (i) solvents and/or (ii) bases or acids.

5. Compound as defined in any of Claims 1 to 3 for the treatment and/or prophylaxis of diseases.

6. Use of a compound as defined in any of Claims 1 to 3 for preparing a medicament for the treatment and/or prophylaxis of angina pectoris, pulmonary hypertension, thromboembolic disorders and peripheral occlusive diseases.

7. Medicament comprising a compound as defined in any of Claims 1 to 3 in combination with an inert nontoxic pharmaceutically acceptable auxiliary.

8. Medicament comprising a compound as defined in any of Claims 1 to 3 in combination with a further active compound.

9. Medicament according to Claim 7 or 8 for the treatment and/or prophylaxis of angina pectoris, pulmonary hypertension, thromboembolic disorders and peripheral occlusive diseases.

## Revendications

1. Composé de formule (I) 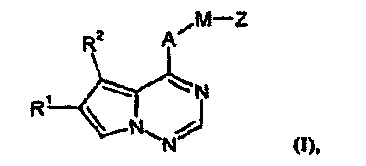 dans laquelle
A représente O ou N-R³, où
R³ signifie hydrogène, (C₁-C₆) -alkyle, (C₃-C₇) - cycloalkyle ou (C₄-C₇)-cycloalcényle,
M représente un groupe de formule 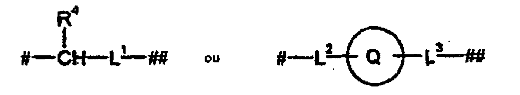 où
# signifie le site de liaison avec le groupe A et
## signifie le site de liaison avec le groupe Z,
R⁴ signifie hydrogène ou (C₁-C₄) -alkyle, qui peut être substitué par hydroxy ou amino,
L¹ signifie (C₁-C₇) -alcanediyle ou (C₂-C₇- alcènediyle, qui peuvent être monosubstitués ou disubstitués par fluor, ou un groupe de formule *-L^{1A}-V-L^{1B}-**, où
* représente le site de liaison avec le groupe -CHR⁴,
** représente le site de liaison avec le groupe Z,
L^{1A} représente (C₁-C₅) -alcanediyle, qui peut être monosubstitué ou disubstitué, de manière identique ou différente, par (C₁-C₄) -alkyle et/ou (C₁-C₄) -alcoxy,
L^{1B} représente une liaison ou (C₁-C₃) - alcanediyle, qui peut être monosubstitué ou disubstitué par fluor, et
V représente O ou N-R⁵, où
R⁵ signifie hydrogène, (C₁-C₆) -alkyle ou (C₃-C₇-cycloalkyle,
L² signifie une liaison ou (C₁-C₄)-alcanediyle,
L³ signifie (C₁-C₄) -alcanediyle, qui peut être monosubstitué ou disubstitué par fluor et dans lequel un groupe méthylène peut être remplacé par O ou N-R⁶, où R⁶ représente hydrogène, (C₁-C₆) -alkyle ou (C₃-C₇-cycloalkyle, ou signifie (C₂-C₄) -alcènediyle, et
Q signifie (C₃-C₇) -cycloalkyle, (C₄-C₇)- cycloalcényle, phényle, hétérocyclyle de 5 à 7 chaînons ou hétéroaryle de 5 ou 6 chaînons, qui peuvent à chaque fois être jusqu'à disubstitués, de manière identique ou différente, par des radicaux choisis dans la série fluor, chlore, (C₁-C₄) -alkyle, trifluorométhyle, hydroxy, (C₁-C₄)-alcoxy, trifluorométhoxy, amino, mono-(C₁-C₄)- alkylamino et di-(C₁-C₄)-alkylamino, où (C₁-C₄) -alkyle peut à son tour être substitué par hydroxy, (C₁-C₄)-alcoxy, amino, mono- (C₁-C₄) -alkylamino ou di- (C₁- C₄)-alkylamino,
Z représente un groupe de formule 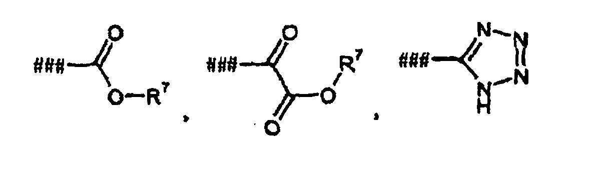 ou 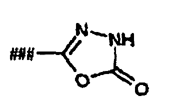 où
### signifie le site de liaison avec le groupe L¹ ou, selon le cas, L³ et
R⁷ signifie hydrogène ou (C₁-C₄) -alkyle, et
R¹ et R² sont identiques ou différentes et représentent, indépendamment l'un de l'autre, (C₃-C₇) - cycloalkyle, (C₄-C₇)-cycloalcényle, phényle, hétérocyclyle de 5 à 7 chaînons ou hétéroaryle de 5 ou 6 chaînons, qui peuvent à chaque fois être monosubstitués à trisubstitués, de manière identique ou différente, par des radicaux choisis dans la série halogène, cyano, nitro, (C₁-C₆)-alkyle, (C₂-C₆) -alcényle, (C₂-C₄) -alcynyle, (C₃-C₇)-cycloalkyle, (C₄-C₇)-cycloalcényle, (C₁-C₆)-alcoxy, trifluorométhyle, trifluorométhoxy, (C₁-C₆) - alkylthio, (C₁-C₆) -acyle, amino, mono- (C₁-C₆) - alkylamino, di- (C₁-C₆) -alkylamino et (C₁-C₆) - acylamino,
où (C₁-C₆) -alkyle et (C₁-C₆) -alcoxy peuvent à leur tour être substitués à chaque fois par cyano, hydroxy, (C₁-C₄) -alcoxy, (C₁-C₄) -alkylthio, amino, mono-(C₁-C₄) -alkylamino ou di-(C₁-C₄) -alkylamino, ou
R¹ et/ou R² représentent phényle, où deux radicaux liés à des atomes de carbone de cycle adjacents forment ensemble un groupe de formule -O-CH₂-O-, -O-CHF-O-, -O-CF₂-O-, -O-CH₂CH₂-O- ou -O-CF₂-CF₂-O-,
ainsi que ses sels, solvates et les solvates des sels.

2. Composé de formule (I) selon la revendication 1, dans laquelle
A représente O ou NH,
M représente un groupe de formule 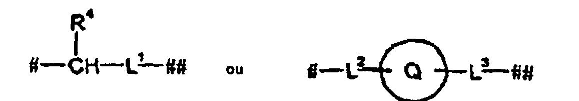 où
# signifie le site de liaison avec le groupe A et
## signifie le site de liaison avec le groupe Z,
R⁴ signifie hydrogène, méthyle ou éthyle,
L¹ signifie (C₃-C₇) -alcanediyle, (C₃-C₇) - alcènediyle ou un groupe de formule *-L^{1A}-V- L_{1B}-**, où
* représente le site de liaison avec le groupe -CHR⁴,
** représente le site de liaison avec le groupe Z,
L^{1A} représente (C₁-C₃) -alcanediyle, qui peut être monosubstitué ou disubstitué par méthyle,
L^{1B} représente (C₁-C₃) -alcanediyle et
V représente O ou N-CH₃
L² signifie une liaison, méthylène, éthane-1,1- diyle ou éthane-1,2-diyle,
L³ signifie (C₁-C₃)-alcanediyle ou un groupe de formule *-W-CH₂-** ou *-W-CH₂-CH₂-*, où
* représente le site de liaison avec le cycle Q,
** représente le site de liaison avec le groupe Z et
W représente O ou N-R⁶, où
R⁶ signifie hydrogène ou (C₁-C₃) - alkyle, et
Q signifie cyclobutyle, cyclopentyle, cyclopentényle, cyclohexyle, pyrrolidinyle, pipéridinyle, tétrahydrofurannyle, tétrahydropyrannyle, morpholinyle ou phényle, qui peuvent à chaque fois être jusqu'à disubstitués, de manière identique ou différente, par des radicaux choisis dans la série fluor, méthyle, éthyle, trifluorométhyle, hydroxy, méthoxy et éthoxy, Z représente un groupe de formule 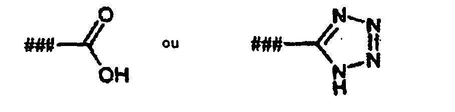 où
### signifie le site de liaison avec le groupe L¹ ou, selon le cas, L³ et
R¹ et R² sont identiques ou différents et représentent, indépendamment l'un de l'autre, cyclopentén-1-yle, cyclohexén-1-yle, phényle, thiényle ou pyridyle, qui peuvent à chaque fois être monosubstitués ou disubstitués, de manière identique ou différente, par des radicaux choisis dans la série fluor, chlore, cyano, (C₁-C₄) -alkyle, (C₂-C₄)alcényle, (C₁-C₄)-alcoxy, trifluorométhyle et trifluorométhoxy,
ainsi que ses sels, solvates et les solvates des sels.

3. Composé de formule (I) selon la revendication 1 ou 2, dans laquelle
A représente O ou NH,
M représente le groupe de formule 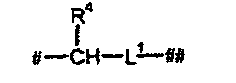 où
# représente le site de liaison avec le groupe A et ## signifie le site de liaison avec le groupe Z,
R⁴ signifie hydrogène ou méthyle, et
L¹ signifie butane-1,4-diyle, pentane-1,5-diyle ou un groupe de formule *-L^{1A}-O-L^{1B}-**, où
* représente le site de liaison avec le groupe - CHR⁴,
** représente le site de liaison avec le groupe
Z,
L^{1A} représente méthylène ou éthane-1,2-diyle, qui peuvent être monosubstitués ou disubstitués par méthyle, et
L^{1B} représente méthylène ou éthane-1,2-diyle Z représente le groupe de formule 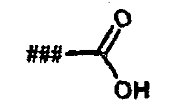 où
### signifie le site de liaison avec le groupe L¹,
R¹ représente phényle, qui peut être substitué par fluor ou chlore, et
R² représente phényle, qui peut être substitué par méthyle, éthyle, méthoxy ou éthoxy,
ainsi que ses sels, solvates et les solvates des sels.

4. Procédé pour la préparation de composés tels que définis dans les revendications 1 à 3, où Z représente -COOH ou -C(=O)-COOH, **caractérisé en ce que** soit
[A] on transforme des composés de formule (II) 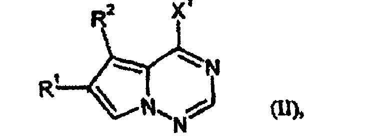 dans laquelle R¹ et R² présentent les significations indiquées dans les revendications 1 à 3 et
X¹ représente un groupe partant tel que par exemple halogène, en particulier chlore, dans un solvant inerte en présence d'une base avec un composé de formule (III) 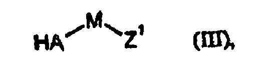 dans laquelle A et M présentent les significations indiquées dans les revendications 1 à 3 et
Z¹ représente cyano ou un groupe de formule - -[C(O)]y-COOR^{7A}, où
y vaut 0 ou 1 et
R^{7A} signifie (C₁-C₄) -alkyle ; en composés de formule (IV) 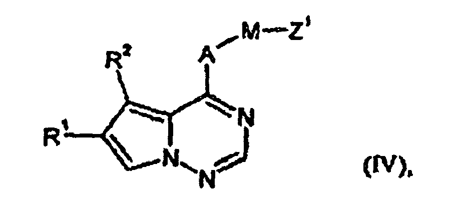 dans laquelle A, M, Z¹, R¹ et R² présentent à chaque fois les significations indiquées ci-dessus, soit
[B] on transforme des composés de formule (V) 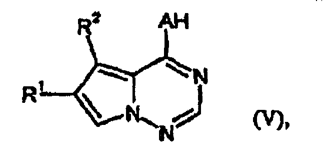 dans laquelle A, R¹ et R² présentent à chaque fois les significations indiquées dans les revendications 1 à 3,
dans un solvant inerte en présence d'une base avec un composé de formule (VI) 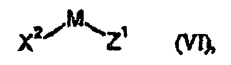 dans laquelle M présente la signification indiquée dans les revendications 1 à 3 et Z¹ la signification indiquée ci-dessus et
X² représente un groupe partant tel que par
exemple halogène, mésylate, tosylate ou triflate,
en composés de formule (IV) 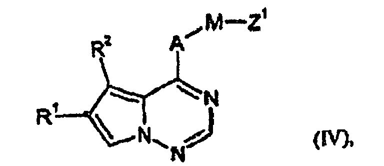 dans laquelle A, M, Z¹, R¹ et R² présentent à chaque fois les significations indiquées ci-dessus,
et on transforme ensuite les composés de formule (IV)
par hydrolyse du groupe ester ou, selon le cas, cyano Z¹ en acides carboxyliques de formule (I-A) 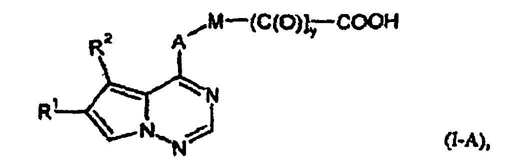 dans laquelle A, M, R¹, R² et y présentent à chaque fois les significations indiquées ci-dessus,
et on transforme ceux-ci le cas échéant avec (i) les solvants et/ou (ii) les bases ou acides correspondants en leurs solvates, sels et/ou solvates des sels.

5. Composé tel que défini dans l'une quelconque des revendications 1 à 3, destiné au traitement et/ou à la prophylaxie de maladies.

6. Utilisation d'un composé, tel que défini selon l'une quelconque des revendications 1 à 3, pour la préparation d'un médicament destiné au traitement et/ou à la prophylaxie de l'angine de poitrine, de l'hypertonie pulmonaire, de maladies thromboemboliques et de maladies occlusives périphériques.

7. Médicament contenant un composé, tel que défini dans l'une quelconque des revendications 1 à 3, en combinaison avec un adjuvant inerte, non toxique, pharmaceutiquement approprié.

8. Médicament contenant un composé tel que défini dans l'une quelconque des revendications 1 à 3 en combinaison avec une autre substance active.

9. Médicament selon la revendication 7 ou 8 destiné au traitement et/ou à la prophylaxie de l'angine de poitrine, de l'hypertonie pulmonaire, de maladies thromboemboliques et de maladies d'occlusion périphériques.
